# EUROPEAN PATENT SPECIFICATION

(11) **EP 3 423 081 B1**
(45) Date of publication and mention of the grant of the patent: **14.07.2021**
(21) Application number: 17707869.8
(22) Date of filing: 28.02.2017
(51) Int. Cl.: A61K 38/22, A61P 25/28

(54) **COMPOSITIONS FOR USE IN THE TREATMENT OF NEUROLOGICAL DISEASE**
ZUSAMMENSETZUNGEN ZUR VERWENDUNG IN DER BEHANDLUNG VON NEUROLOGISCHEN ERKRANKUNGEN
COMPOSITIONS À UTILISER DANS LE TRAITEMENT D'UNE MALADIE NEUROLOGIQUE

(30) Priority: 29.02.2016 GB 201603510
(43) Date of publication of application: 09.01.2019
(73) Proprietor: University Of Ulster, County Londonderry BT52 1SA (GB)
(72) Inventor: MCCLEAN, Paula, Enagh Derry BT47 6WD (GB)
(74) Representative: FRKelly
(86) International application number: PCT/EP2017/054665
(87) International publication number: WO 2017/148960

(56) References cited:
- EP-A1- 2 997 966
- WO-A1-2013/104929
- WO-A2-2009/030498
- US-A1- 2015 164 995
- V. A. GAULT ET AL: "Xenin-25[Lys13PAL]: a novel long-acting acylated analogue of xenin-25 with promising antidiabetic potential", ACTA DIABETOLOGICA., vol. 52, no. 3, 6 November 2014 (2014-11-06), pages 461-471, XP055366839, DE ISSN: 0940-5429, DOI: 10.1007/s00592-014-0681-0
- GAULT VICTOR A ET AL: "Protease-resistant glucose-dependent insulinotropic polypeptide agonists facilitate hippocampal LTP and reverse the impairment of LTP induced by beta-amyloid", JOURNAL OF NEUROPHYSIOLOGY, AMERICAN PHYSIOLOGICAL SOCIETY, BETHESDA, MD, US, vol. 99, no. 4, 1 April 2008 (2008-04-01), pages 1590-1595, XP009111976, ISSN: 0022-3077
- NIGEL IRWIN ET AL: "New perspectives on exploitation of incretin peptides for the treatment of diabetes and related disorders", WORLD JOURNAL OF DIABETES, vol. 6, no. 15, 1 January 2015 (2015-01-01), page 1285, XP055367003, ISSN: 1948-9358, DOI: 10.4239/wjd.v6.i15.1285

## Description

### Background to the Invention

Alzheimer's disease (AD) is a chronic, degenerative disease of the brain which currently affects 44 million people worldwide, with one new case being diagnosed every 3.2 seconds. The disease is a neurodegenerative condition characterised by the accumulation of amyloid-β plaques, significant neuroinflammation, deterioration of synapse structure and function and cognitive decline. The incidence of disease and other forms of dementia is expected to continue to grow significantly with an estimated 100 million people impacted worldwide by 2050.

A recent study published in the medical journal of the American Academy of Neurology has pinned AD as the third leading cause of death in the US and the global cost of the disease is estimated to be 1% of the world's GDP (USD605 billion).

Development of treatments for AD has been notoriously slow and only 5 drugs are currently marketed that offer only symptomatic relief. Treatment for AD is currently limited to these five drugs, which are from two different classes (cholinesterase inhibitors and NMDA receptor antagonists). The complex, multifactorial pathophysiology of AD means that even currently marketed drugs possess limited and inconsistent efficacy. Cholinesterase inhibitors, such as donepezil, offer only symptomatic relief and clinical trials in mild cognitive impairment (MCI) patients have shown that cholinesterase inhibitors fail to halt the progression to AD.

Clinical trials aimed at developing treatments based on the predictions of the amyloid cascade hypothesis have also been largely unsuccessful. Amyloid-β (Aβ) immunisation is thought to activate microglial phagocytosis of Aβ or directly neutralise toxic Aβ oligomers and reduces or prevents development of amyloid plaques in preclinical models. Clinical trials however have suggested that active or passive immunisation has limited therapeutic efficacy in AD patients and may also produce harmful side effects.

While immunotherapy enhances the immune system in order to clear Aβ, it is well known that neuroinflammation contributes to the pathophysiology of AD. Therefore, suppression of the immune system has been considered as an alternative therapeutic approach in AD. It has been observed that non-steroidal anti-inflammatory drug (NSAID) use considerably reduces the likelihood of developing AD, but debate is ongoing as to the precise nature of the relationship between NSAID use and AD risk and although observational and longitudinal, population-based studies continue to suggest a protective effect, randomised controlled trials testing NSAIDs as an intervention in AD patients have mostly been unsuccessful.

Inhibitors of γ-secretase have been shown to influence APP processing and reduce Aβ production in vivo and in vitro, along with restoration of memory function in a cognitively impaired mouse model of AD. However, several clinical trials testing γ-secretase inhibitors in AD patients have either been halted due to serious adverse effects, shown limited efficacy, or both. Similarly, while inhibition of β-secretase shows promise as a modulator of AD pathology in preclinical models, clinical trial success for β-secretase inhibitors is relatively scarce. In any case, β- and γ-secretase enzymes are essential for normal physiological functioning of the CNS, and amyloid precursor protein (APP) is only one of a number of secretase substrates and it is clear that these enzymes have several important physiological roles. This could explain the disappointing results of clinical trials of secretase inhibitors. The serious adverse effects and limited efficacy demonstrated in these trials suggests that β- and γ-secretase inhibitors may have limited clinical application. Although there is evidence that indicates Aβ production can be suppressed in the CNS through modulation of secretase enzymes, inhibiting β- and γ-secretase may not be a feasible therapeutic approach.

Taken together, existing therapies only provide for symptomatic treatment and there are few drugs in development which offer promise. As such, there is a clear and unmet need for development of new therapies possessing disease-modifying abilities and differentiated mechanisms of action to combat this devastating disease.

Biological peptides are one of the most attractive and emerging candidates currently being developed for a host of therapeutic indications due to their numerous advantages over small molecules and/or biologics including high potency, low toxicity, high specificity, low risk of organ accumulation and drug-drug interaction.

### Summary of the Invention

The invention is defined in the claims.

According to a first aspect of the present invention, there is provided a peptide analogue of xenin for use in the treatment of neurological disorders, wherein the peptide analogue comprises xenin and at least one amino acid substitution or modification comprising addition of a fatty acid selected from the group comprising a C-8 octanoyl group, a C-10 decanoyl group, a C-12 lauroyl group, a C-14 myristoyl group, a C-16 palmitoyl group, a C-18 stearoyl group, and a C-20 acyl group.

Optionally, the peptide analogue is a peptide analogue of human xenin.

Optionally, the peptide analogue is an analogue of xenin having the GenBank Accession No AAB23907.

Further optionally, the peptide analogue is an analogue of xenin having the GenBank Accession No AAB23907.1 .

Optionally, the peptide analogue is an analogue of xenin having the amino acid sequence MLTKFETKSARVKGLSFHPKRPWIL.

The peptide analogue comprises xenin and at least one amino acid substitution or modification.

The peptide analogue comprises xenin and at least one amino acid modification.

The at least one amino acid modification comprises addition of a fatty acid selected from the group comprising a C-8 octanoyl group, a C-10 decanoyl group, a C-12 lauroyl group, a C-14 myristoyl group, a C-16 palmitoyl group, a C-18 stearoyl group, and a C-20 acyl group.

Optionally, the at least one amino acid modification comprises the addition of a C-16 palmitoyl group.

Optionally, the peptide analogue comprises xenin and a C-16 palmitoyl group.

Optionally, the or each fatty acid is attached adjacent or at the N-terminal amino acid residue of the peptide analogue; adjacent or at the C-terminal amino acid residue of the peptide analogue; or at any other amino acid residue of the amino acid sequence of the peptide analogue.

Optionally, the or each fatty acid is attached at a lysine residue.

Further optionally, the or each fatty acid is attached at an alpha or epsilon amino group of a lysine residue.

Optionally, the peptide analogue comprises xenin and a C-16 palmitoyl group attached at a lysine residue.

Further optionally, the peptide analogue comprises xenin and a C-16 palmitoyl group attached at the lysine residue at position 13 of the peptide analogue.

Disclosed herein, there is provided a peptide analogue of gastric inhibitory polypeptide, or a fragment thereof, for use in the treatment of neurological disorders.

Optionally, the peptide analogue is a peptide analogue of human gastric inhibitory polypeptide, or a fragment thereof.

Optionally, the peptide analogue is an analogue of gastric inhibitory polypeptide having the GenBank Accession No AAA88043, or a fragment thereof.

Further optionally, the peptide analogue is an analogue of gastric inhibitory polypeptide having the GenBank Accession No AAA88043.1, or a fragment thereof.

Optionally, the peptide analogue is an analogue of gastric inhibitory polypeptide having the amino acid sequence
MVATKTFALLLLSLFLAVGLGEKKEGHFSALPSLPVGSHAKVSSPQPRGPRYAEGTFISDYSIAMDKI HQQDFVNWLLAQKGKKNDWKHNITQREARALELASQANRKEEEAVEPQSSPAKNPSDEDLLRDLLI QELLACLLDQTNLCRLRSR, or a fragment thereof.

Optionally, the peptide analogue is an analogue of a fragment of gastric inhibitory polypeptide comprising amino acid residues at positions 52 - 93 of the amino acid sequence

Further optionally, the peptide analogue is an analogue of a fragment of gastric inhibitory polypeptide, the fragment comprising the amino acid sequence
YAEGTFISDYSIAMDKIHQQDFVNWLLAQKGKKNDWKHNITQ.

Further optionally, the peptide analogue is an analogue comprising at least 12 amino acid residues from the N-terminal end of gastric inhibitory polypeptide having the amino acid sequence
YAEGTFISDYSIAMDKIHQQDFVNWLLAQKGKKNDWKHNITQ.

Optionally, the peptide analogue is an analogue of gastric inhibitory polypeptide having the amino acid sequence YAEGTFISDYSIAMDKIHQQDFVNWLLAQKGKKNDWKHNITQ.

Optionally, the peptide analogue comprises gastric inhibitory polypeptide and at least one amino acid substitution or modification.

Optionally, the peptide analogue comprises gastric inhibitory polypeptide and at least one amino acid modification.

Optionally, the at least one amino acid modification comprises acylation of an amino acid residue.

Optionally, the at least one amino acid modification comprises acetylation of an amino acid residue.

Further optionally, the at least one amino acid modification comprises acetylation of the N-terminal amino acid residue of the peptide analogue; acetylation of the C-terminal amino acid residue of the peptide analogue; or acetylation of any other amino acid residue of the amino acid sequence of the peptide analogue.

Still further optionally, the at least one amino acid modification comprises acetylation of the N-terminal amino acid residue of the peptide analogue.

Optionally, the peptide analogue comprises gastric inhibitory polypeptide, wherein the N-terminal amino acid residue of the peptide analogue is acetylated.

Optionally, the peptide analogue further comprises at least one amino acid modification comprising addition of a fatty acid selected from the group comprising a C-8 octanoyl group, a C-10 decanoyl group, a C-12 lauroyl group, a C-14 myristoyl group, a C-16 palmitoyl group, a C-18 stearoyl group, and a C-20 acyl group.

Further optionally, the at least one amino acid modification comprises the addition of a C-16 palmitoyl group.

Optionally, the peptide analogue comprises gastric inhibitory polypeptide and a C-16 palmitoyl group. Further optionally, the peptide analogue comprises gastric inhibitory polypeptide wherein the N-terminal amino acid residue of the peptide analogue is acetylated, and a C-16 palmitoyl group.

Optionally, the or each fatty acid is attached adjacent or at the N-terminal amino acid residue of the peptide analogue; adjacent or at the C-terminal amino acid residue of the peptide analogue; or at any other amino acid residue of the amino acid sequence of the peptide analogue.

Optionally, the or each fatty acid is attached to a lysine residue.

Further optionally, the or each fatty acid is attached to an alpha or epsilon amino group of a lysine residue.

Optionally, the peptide analogue comprises gastric inhibitory polypeptide and a C-16 palmitoyl group attached to a lysine residue. Further optionally, the peptide analogue comprises gastric inhibitory polypeptide wherein the N-terminal amino acid residue of the peptide analogue is acetylated, and a C-16 palmitoyl group attached to a lysine residue.

Further optionally, the peptide analogue comprises gastric inhibitory polypeptide and a C-16 palmitoyl group attached to the lysine residue at position 37 of the peptide analogue. Further optionally, the peptide analogue comprises gastric inhibitory polypeptide wherein the N-terminal amino acid residue of the peptide analogue is acetylated, and a C-16 palmitoyl group attached to the lysine residue at position 37 of the peptide analogue.

Further optionally, the peptide analogue comprises gastric inhibitory polypeptide and a C-16 palmitoyl group attached to the lysine residue at position 37 of the peptide analogue. Further optionally, the peptide analogue comprises gastric inhibitory polypeptide wherein the N-terminal amino acid residue of the peptide analogue is acetylated, and a C-16 palmitoyl group attached to the lysine residue at position 37 of the peptide analogue having the amino acid sequence
YAEGTFISDYSIAMDKIHQQDFVNWLLAQKGKKNDWKHNITQ.

Optionally, the peptide analogue further comprises at least one amino acid modification comprising attachment of a polymer moiety of the general formula HO-(CH2-O-CH2)n-H, wherein n is an integer between 1 and 22.

Further optionally, the peptide analogue comprises xenin and at least one amino acid substitution or modification, and the peptide analogue further comprises at least one amino acid modification comprising attachment of a polymer moiety of the general formula HO-(CH2-O-CH2)n-H, wherein n is an integer between 1 and 22.

Alternatively, the peptide analogue comprises gastric inhibitory polypeptide or a fragment thereof and at least one amino acid substitution or modification, and the peptide analogue further comprises at least one amino acid modification comprising attachment of a polymer moiety of the general formula HO-(CH2-O-CH2)n-H, wherein n is an integer between 1 and 22.

Optionally, n is an integer between 1 and about 10.

Further optionally, n is an integer between about 2 and about 5.

Optionally, the polymer moiety has a branched structure. The branched structure may comprise the attachment of at least two polymer moieties of linear structure.

Alternatively, the branch point may be located within the structure of each polymer moiety.

Alternatively, the polymer moiety has a linear structure.

Optionally, the or each polymer moiety is attached adjacent or at the N-terminal amino acid residue of the peptide analogue; adjacent or at the C-terminal amino acid residue of the peptide analogue; or at any other amino acid residue of the amino acid sequence of the peptide analogue.

Optionally, the or each polymer moiety is attached adjacent or at the C-terminal amino acid residue.

Further optionally, the or each polymer moiety is attached at the C-terminal amino acid residue.

Optionally, the or each polymer moiety is attached at a lysine residue.

Further optionally, the or each polymer moiety is attached at an alpha or epsilon amino group of a lysine residue.

According to a second aspect of the present invention, there is provided a composition comprising a peptide analogue according to a first aspect of the invention and a pharmaceutically acceptable carrier.

Optionally, the composition comprises a pharmaceutically effective amount of the peptide analogue according to a first aspect of the invention and a pharmaceutically acceptable carrier.

Optionally, use comprises administration of a peptide analogue according to a first aspect of the invention or a composition according to a second aspect of the invention.

Further optionally, use comprises administration of a peptide analogue according to a first aspect of the invention or a composition according to a second aspect of the invention to a subject.

Further optionally, use comprises administration of a peptide analogue according to a first aspect of the invention or a composition according to a second aspect of the invention to a subject suffering from a neurological disorder.

Further optionally, use comprises administration of a pharmaceutically effective amount of a peptide analogue according to a first aspect of the invention or a composition according to a second aspect of the invention.

Still further optionally, use comprises administration of 0.25 - 25.00 nmol/kg body weight of a peptide analogue according to a first aspect of the invention or an equivalent amount of a composition according to a second aspect of the invention.

Still further optionally, use comprises administration of 2.50 - 25.00 nmol/kg body weight of a peptide analogue according to a first aspect of the invention or an equivalent amount of a composition according to a second aspect of the invention.

Still further optionally, use comprises administration of 25.00 nmol/kg body weight of a peptide analogue according to a first aspect of the invention or an equivalent amount of a composition according to a second aspect of the invention.
Disclosed herein, there is provided a method for treating neurological disorders, the method comprising administering a peptide analogue according to a first or second aspect of the invention or a composition according to a third aspect of the invention.

Disclosed herein, there is provided use of a peptide analogue according to a first or second aspect of the invention in the manufacture of a medicament for treating neurological disorders.

Optionally, the neurological disorder is a disorder selected from the group of disorders affecting cognitive function; and dysfunctional cognitive processes.

Optionally, disorders negatively affecting cognitive function include, but are not limited to: dementia, stroke, schizophrenia, bipolar disorder, and neurodegenerative diseases.

Optionally, disorders positively affecting cognitive function include, but are not limited to: post-traumatic stress disorder, epilepsy, Tourette's syndrome, and hallucinations.

Optionally, neurodegenerative diseases are selected from, but not limited to: Alzheimer's disease (AD), Creutzfeldt-Jacob disease (CJD), Huntington's disease, and Parkinson's disease.

Optionally, dysfunctional cognitive processes include, but are not limited to: attention, calculation, memory, judgment, insight, learning, and reasoning.

Optionally, the neurological disorder is Alzheimer's disease.

Optionally, the peptide analogue is for use in improving cognitive function.

Optionally or additionally, the peptide analogue is for use in improving memory. Further optionally or additionally, the peptide analogue is for use in improving recognition or spatial memory.

Optionally, the peptide analogue is for use in reducing amyloid beta, optionally for use in reducing amyloid beta dense core plaque number.

Optionally, the peptide analogue is for use in reducing inflammation. Further optionally, the peptide analogue is for use in reducing inflammation in the brain.

Optionally, the peptide analogue is for use in increasing synapse density or number.

Optionally, the peptide analogue is for use in increasing neurogenesis.

### Brief Description of the Drawings

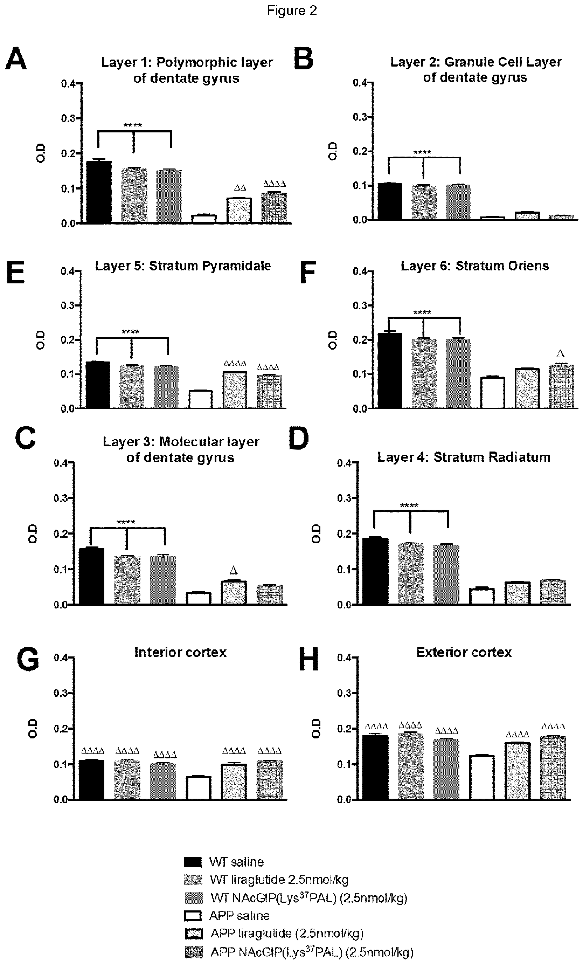

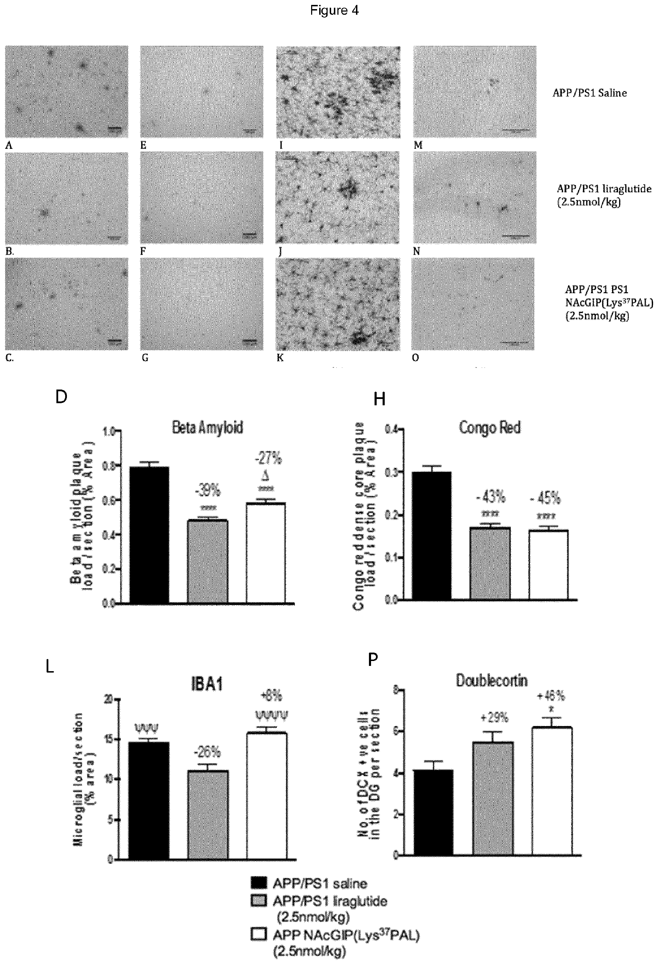

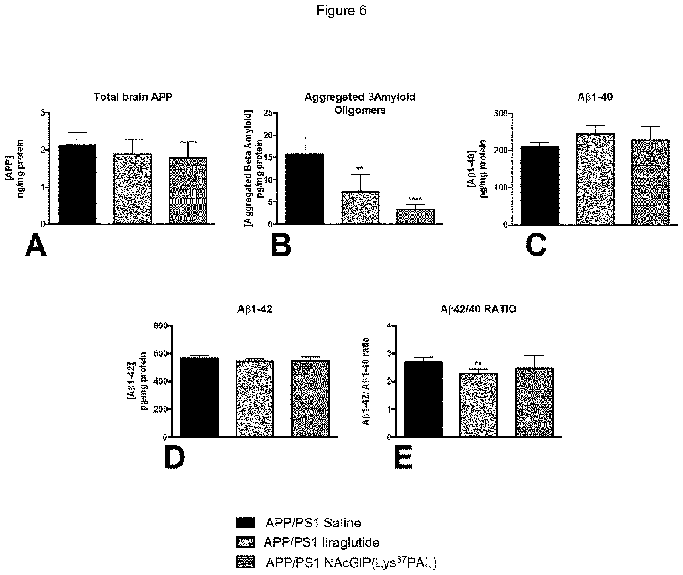

Embodiments of the present invention will now be described with reference to the accompanying drawings in which:

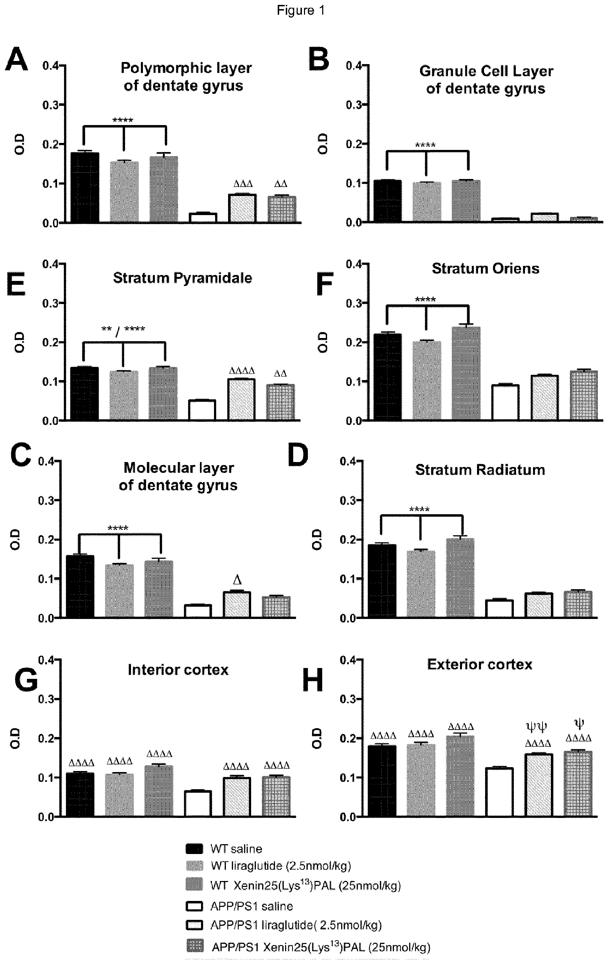

**Figure 1** is a graph illustrating synaptic density is partially preserved by Xenin-25[Lys(13)PAL] and liraglutide in APP/PS1 mice. Synaptophysin levels were measured in wild-type and APP/PS1 mice treated with saline, liraglutide and Xenin-25[Lys(13)PAL] once daily for 10 weeks. Illustrated is quantification of the Polymorphic layer (A), Granule Cell Layer (B), Molecular Layer (C), Stratum Radiatum (D), Stratum Pyrimidale (E), Stratum Oriens of (F), interior (G) and exterior cortex (H). Data representative of mean ± SEM for 8-12 sections for n=6 mice per group.**/****p<0.01-p<0.0001 compared with all groups of APP/PS1 mice. Δp<0.05, ΔΔp<0.01, ΔΔΔp<0.001 and ΔΔΔΔp<0.0001 compared with saline-treated APP/PS1 mice. ψp<0.05 and ψψp<0.01 compared with Xenin-25[Lys(13)PAL] treated wild-type mice;
**Reference** **Figure 2** is a graph illustrating synaptic density is partially preserved by NAcGIP[Lys(37)PAL] and liraglutide in APP/PS1 mice. Synaptophysin levels were measured in wild-type and APP/PS1 mice treated with saline, liraglutide and NAcGIP[Lys(37)PAL] once daily for 10 weeks. Illustrated is quantification of the Polymorphic layer (A), Granule Cell Layer (B), Molecular Layer (C), Stratum Radiatum (D), Stratum Pyrimidale (E), Stratum Oriens of (F), interior (G) and exterior cortex (H). Data representative of mean ± SEM for 8-12 sections for n=6 mice per group. ****P<0.0001 vs. all APP/PS1 groups. Δp<0.05, ΔΔp<0.01 and ΔΔΔΔp<0.0001 compared with saline-treated APP/PS1 mice;

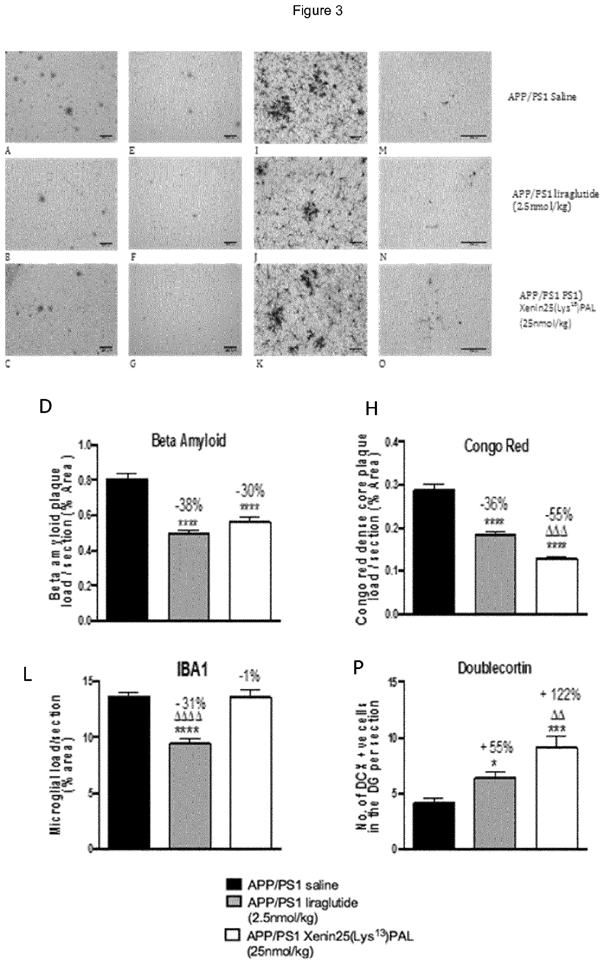

**Figure 3** is a graph illustrating protective effects of Xenin-25[Lys(13)PAL] on key biomarkers in APP/PS1 mouse model. Representative images of beta amyloid plaque load in saline (A), liraglutide (B), and Xenin-25[Lys(13)PAL] treated APP/PS1 mice (C) and quantification in n=6 animals per group (D). Representative images of dense core congo red plaque load in saline (E), liraglutide (F), and Xenin-25[Lys(13)PAL] treated APP/PS1 mice (G) and quantification in n=6 animals per group (H). Representative images of IBA-1 staining in saline (I), liraglutide (J), and Xenin-25[Lys(13)PAL] treated APP/PS1 mice (K) and quantification in n=6 animals per group (L). Representative images of doublecortin staining, a marker of neurogenesis, in saline (M), liraglutide (N), and Xenin-25[Lys(13)PAL] treated APP/PS1 mice (O) and quantification in n=6 animals per group (P). Data representative of mean ± SEM for 8-12 sections for n=6 mice per group.*p<0.05, ***p<0.001 and ****p<0.0001 vs. saline-treated APP/PS1 mice. ΔΔp<0.001, ΔΔΔp<0.001 and ΔΔΔΔp<0.0001 vs. liraglutide-treated APP/PS1 mice;
**Reference** **Figure 4** is a graph illustrating protective effects of NAcGIP[Lys(37)PAL] on key biomarkers in APP/PS1 mouse model. Representative images of beta amyloid plaque load in saline (A), liraglutide (B), and NAcGIP[Lys(37)PAL]-treated APP/PS1 mice (C) and quantification in n=6 animals per group (D). Representative images of dense core congo red plaque load in saline (E), liraglutide (F), and NAcGIP[Lys(37)PAL]treated APP/PS1 mice (G) and quantification in n=6 animals per group (H). Representative images of doublecortin staining, a marker of neurogenesis, in saline (I), liraglutide (J), and NAcGIP[Lys(37)PAL]-treated APP/PS1 mice (K) and quantification in n=6 animals per group (L). Data representative of mean ± SEM for 8-12 sections for n=6 mice per group. *p<0.05 and ****p<0.0001 vs. saline treated APP/PS1 mice. ΔΔp<0.001, ΔΔΔp<0.001. Δp<0.05 vs. liraglutide treated APP/PS1 mice;

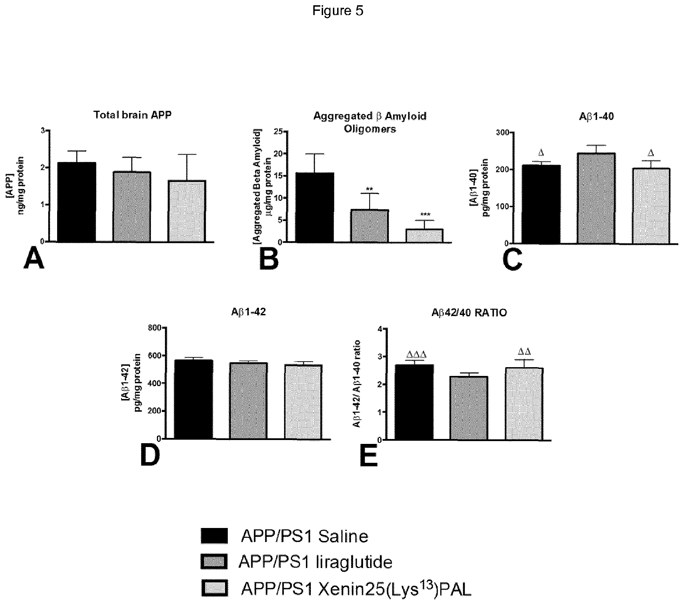

**Figure 5** is a graph illustrating reduction in soluble oligomer levels. After 10 weeks treatment with saline, liraglutide, or Xenin-25[Lys(13)PAL], total brain APP (A), Aggregated beta amyloid oligomers (B), Aβ1-40 (C), Aβ1-42 (D) and Aβ42/40 ratio (E) were measured by ELISA. Values represent mean ± SEM for n=6 brains per group **p<0.01 and ***p<0.001 vs. saline treated APP/PS1 mice. Δp<0.05, ΔΔp<0.01 and ΔΔΔp<0.001 vs. liraglutide-treated APP/PS1 mice;
**Reference** **Figure 6** is a graph illustrating reduction in beta amyloid oligomer levels. After 10 weeks treatment with saline, liraglutide, or NAcGIP(Lys³⁷PAL), total brain APP (A), Aggregated beta amyloid oligomers (B), Aβ1-40 (C), Aβ1-42 (D) and Aβ42/40 ratio (E) were measured by ELISA. Values represent mean ± SEM for n=6 brains per group **p<0.01 and ***p<0.001 vs. saline treated APP/PS1 mice. Δp<0.05, ΔΔp<0.01 and ΔΔΔp<0.001 vs. liraglutide treated APP/PS1 mice;

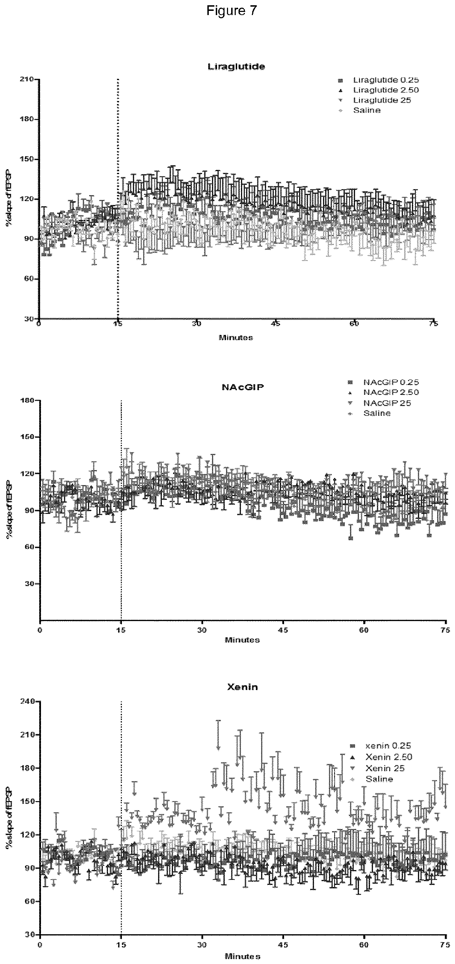

**Figure 7** is an analysis of post high frequency stimulation baselines demonstrating that liraglutide (A) injected once-daily for 3 weeks potentiated the induction and maintenance of LTP in the CA1 of the hippocampus in C57BI/6 mice when compared with saline-treated mice at 0.25, 2.5 and 25nmol/kg, with superior effects observed at lower doses. n=8 per group; N-AcGIP(Lys³⁷PAL) (B) potentiated the induction and maintenance of LTP at 2.5 and 25nmol/kg, while it inhibited the induction of LTP at 0.25nmol/kg; and xenin25(Lys¹³PAL) potentiated the induction and maintenance of LTP at 25nmol/kg, while it inhibited the induction of LTP at 2.5 and 0.25nmol/kg;

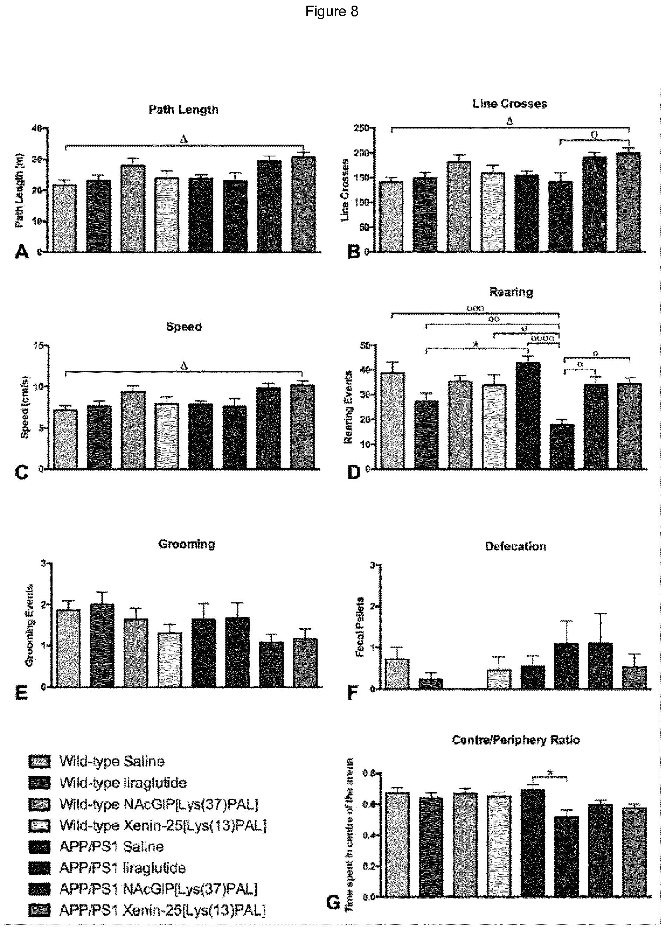

**Figure 8** is a graph illustrating baseline performance in an open field task following acute administration of saline, liraglutide, NAcGIP[Lys(37)PAL] or Xenin-25[Lys(13)PAL] in wild-type and APP/PS1 mice. In an open field task, path length (A), line crosses (B), speed (C), rearing (D), grooming (E), fecal pellets (F) and time spent in the centre of the arena (G) was measured 30 minutes after an acute injection of each of the respective treatments in wild-type and APP/PS1 mice. Δp<0.05 vs. wild-type saline; *p<0.05 vs. APP/PS1 Saline; op<0.05, oop<0.01, ooop<0.001, oooop<0.0001 vs. APP/PS1 liraglutide. Data represent mean ± SEM for 11-13 mice per group;

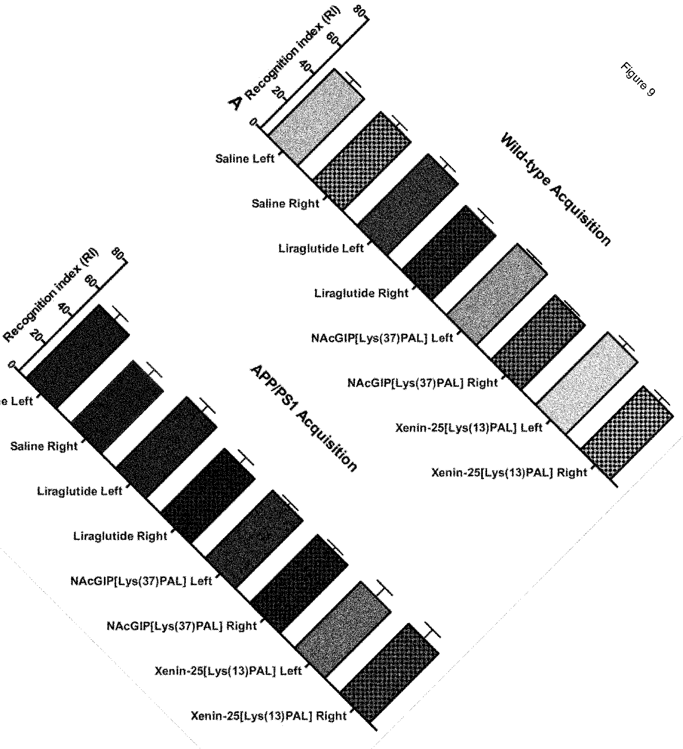

**Figure 9** is a graph illustrating baseline performance in the acquisition phase of the novel object recognition task following acute administration of saline, liraglutide, NAcGIP[Lys(37)PAL] or Xenin-25[Lys(13)PAL] in wild-type and APP/PS1 mice. Twenty-four hours following exposure to the open field arena, a novel object recognition task was conducted using the same arena in wild-type and APP/PS1 mice, 30 minutes after an injection of each of the treatments, outlined above. Either wild-type (A) or APP/PS1 (B) mice were exposed to two identical objects, either white balls or red cubes, in a 10 minute acquisition phase. Data represent mean ± SEM for 11-13 mice per group;

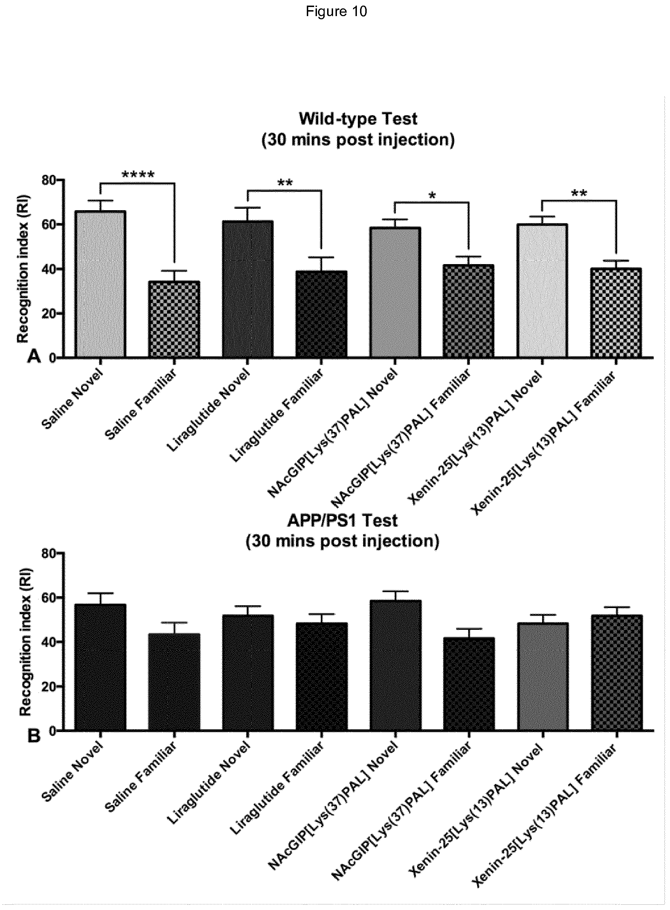

**Figure 10** is a graph illustrating baseline novel object recognition memory following acute administration of saline, liraglutide, NAcGIP[Lys(37)PAL] or Xenin-25[Lys(13)PAL] in wild-type and APP/PS1 mice. The test phase of the novel object recognition task took place 3 hours following the acquisition phase. In the test phase, one of the objects from the acquisition phase was replaced with a novel object, not previously encountered. Illustrated are recognition indices for the familiar, compared with the novel object in wild-type (A) and APP/PS1 (B) mice in the test phase following acute administration of respective treatments. *p<0.05, **p<0.01 and ****p<0.0001 vs. novel object. Data represent mean ± SEM for 11-13 mice per group;

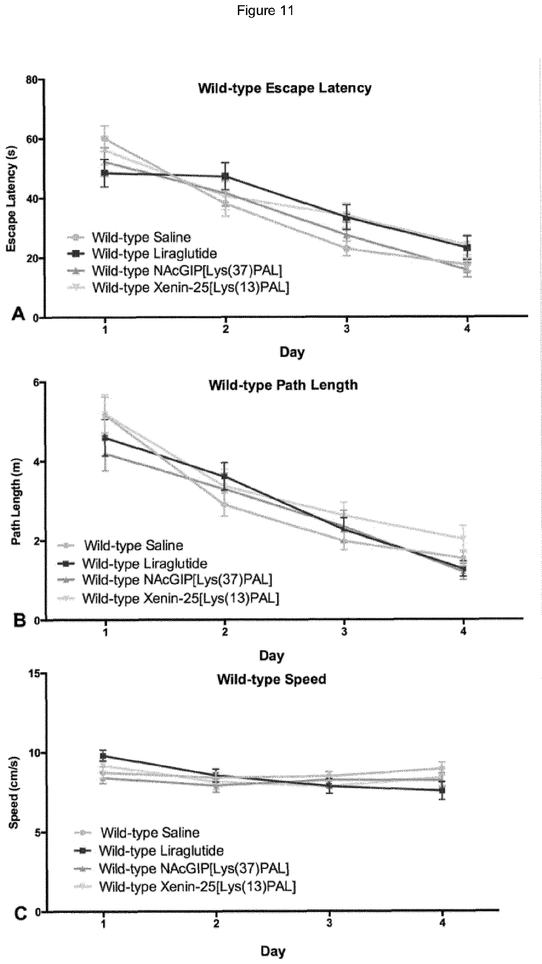

**Figure 11** is a graph illustrating the effects of chronic treatment with liraglutide, NAcGIP[Lys(37)PAL] and Xenin-25[Lys(13)PAL] on spatial learning in wild-type mice. Morris water maze training was initiated after 56 days treatment with saline, liraglutide, NAcGIP[Lys(37)PAL] and Xenin-25[Lys(13)PAL]. The acquisition phase involved 4 training sessions per day over 4 consecutive days. Illustrated is the average escape latency per training day (A), path length (B), and swim speed (C). Data represent mean ± SEM for 11-12 mice per group;

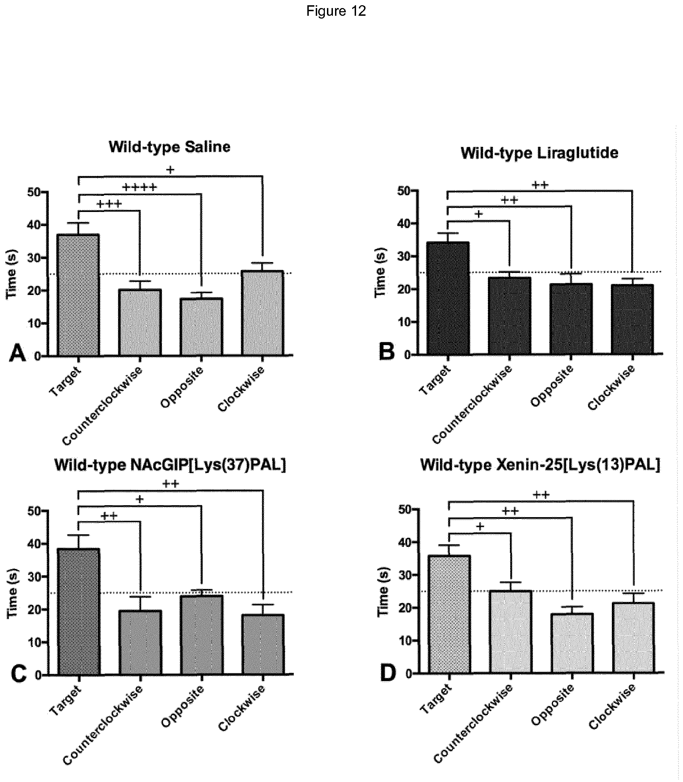

**Figure 12** is a graph illustrating the effects of chronic treatment with liraglutide, NAcGIP[Lys(37)PAL] and Xenin-25[Lys(13)PAL] on spatial memory in wild-type mice. A probe trial was conducted on the fifth day, 24 hours following the final training session of the Morris water maze as a measure of spatial memory. Illustrated above is time spent in each of the quadrants during the probe trial by saline (A), liraglutide (B), NAcGIP[Lys(37)PAL] (C), and Xenin-25[Lys(13)PAL] (D) -treated wild-type mice, following 60 days treatment with each of the respective drugs. +p<0.05, ++p<0.01, +++p<0.001 and ++++p<0.0001 vs. target quadrant. Data represent mean ± SEM for 11-12 mice per group;

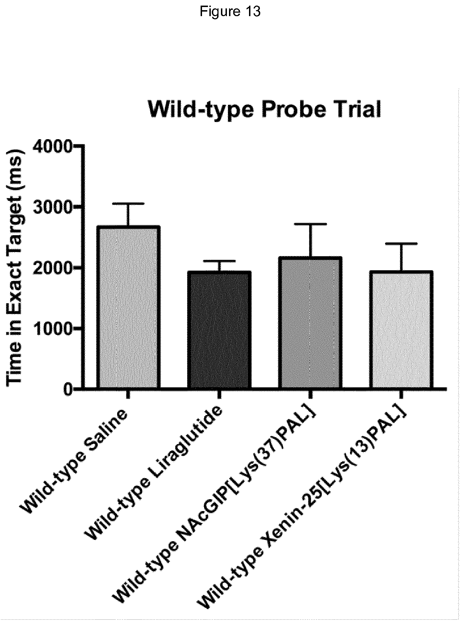

**Figure 13** is a graph illustrating spatial acuity following chronic treatment with liraglutide, NAcGIP[Lys(37)PAL] and Xenin-25[Lys(13)PAL] in wild-type mice. Illustrated above is the amount of time spent within a 1 cm radius of the previous location of the escape platform during the probe trial, which took place 24 hours following the final Morris water maze training sessions, as an indication of spatial acuity in wild-type mice following 60 days treatment with respective drugs. Data represent mean ± SEM for 11-12 mice per group;

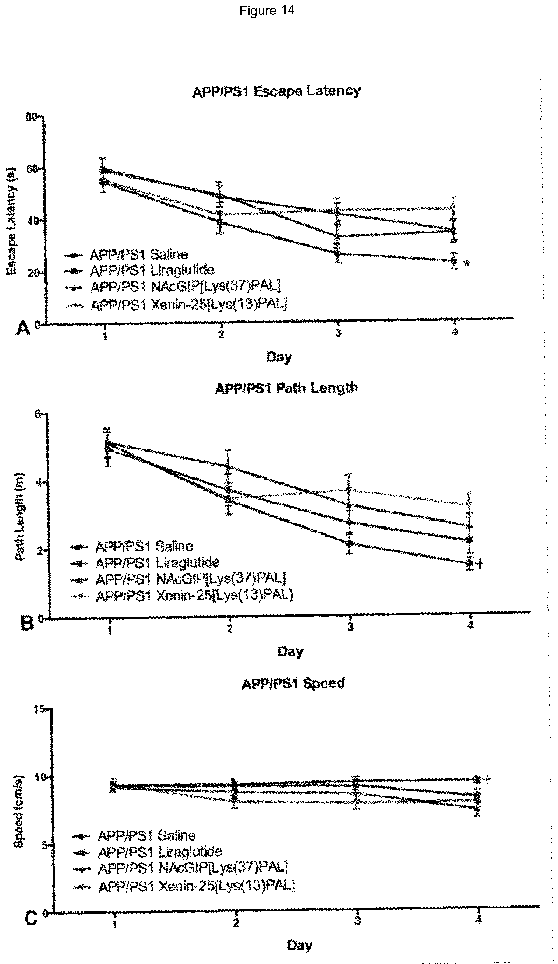

**Figure 14** is a graph illustrating the effects of chronic treatment with liraglutide, NAcGIP[Lys(37)PAL] and Xenin-25[Lys(13)PAL] on spatial learning in APP/PS1 mice. Morris water maze training was initiated after 56 days treatment with saline, liraglutide, NAcGIP[Lys(37)PAL] and Xenin-25[Lys(13)PAL]. The acquisition phase involved 4 training sessions per day over 4 consecutive days. Illustrated is the average escape latency per training day (A), path length to the escape platform (B), and swim speed (C) of APP/PS1 mice following chronic treatment with respective drugs. *p<0.05 vs. APP/PS1 Saline; +p<0.05 vs. APP/PS1 Xenin-25[Lys(13)PAL]. Data represent mean ± SEM for 11-12 mice per group;

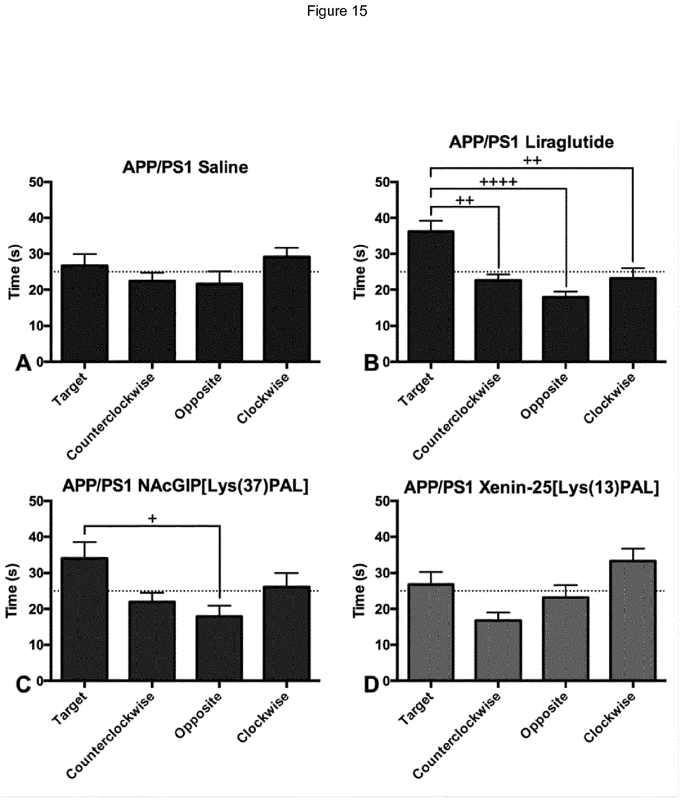

**Figure 15** is a graph illustrating the effects of chronic treatment with liraglutide, NAcGIP[Lys(37)PAL] and Xenin-25[Lys(13)PAL] on spatial memory in APP/PS1 mice. The probe trial took place 24 hours following the final Morris water maze training session as a measurement of spatial memory. Illustrated above is the amount of time spent in each quadrant by saline (A), liraglutide (B), NAcGIP[Lys(37)PAL] (C), and Xenin-25[Lys(13)PAL] (D) -treated APP/PS1 mice, following 60 days of treatment. +p<0.05, ++p<0.01 vs. target quadrant. Data represent mean ± SEM for 11-12 mice per group;

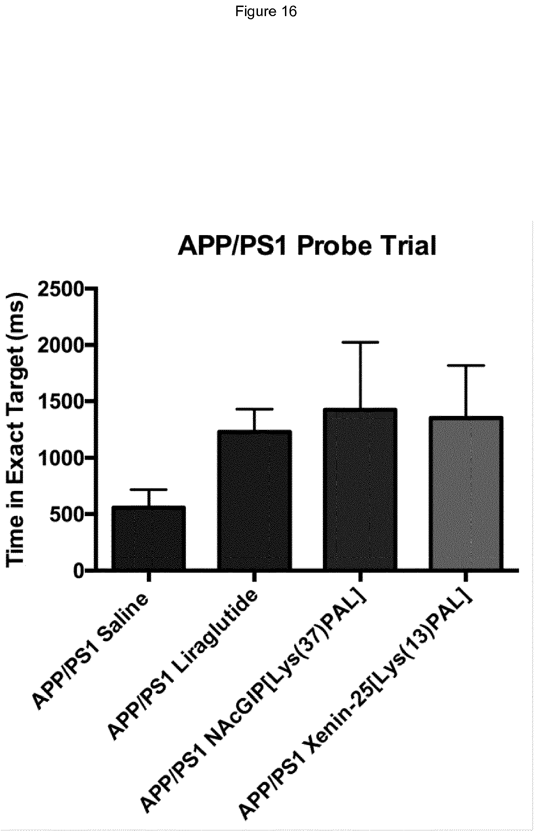

**Figure 16** is a graph illustrating the effects of chronic treatment with liraglutide, NAcGIP[Lys(37)PAL] and Xenin-25[Lys(13)PAL] on spatial acuity in APP/PS1 mice. Illustrated above is the amount of time spent within a 1 cm radius of the previous location of the escape platform during the probe trial, which took place 24 hours following the final Morris water maze training session, as an indication of spatial acuity in APP/PS1 mice following 60 days treatment with respective drugs. Data represent mean ± SEM for 11-12 mice per group;

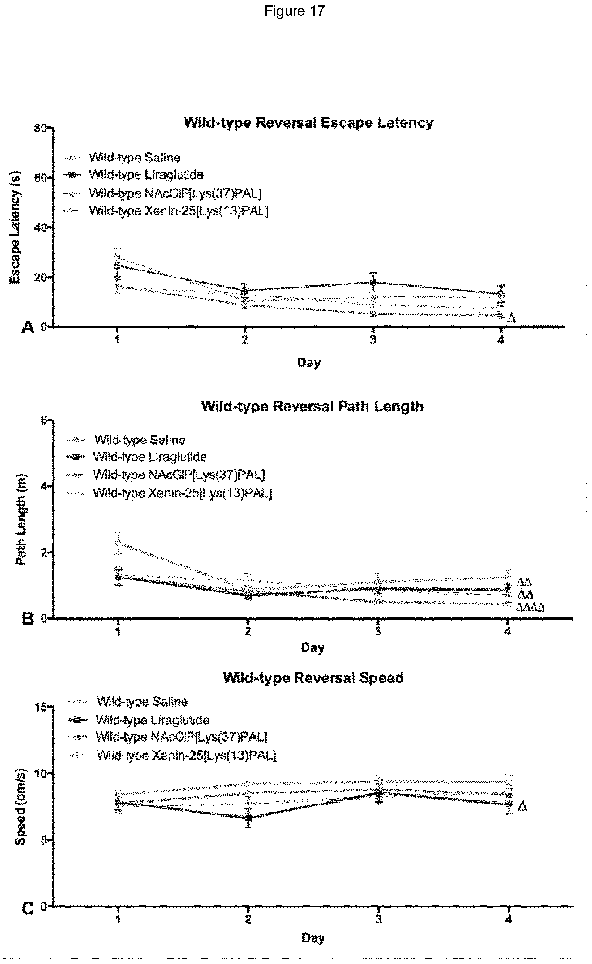

**Figure 17** is a graph illustrating the effects of chronic treatment with liraglutide, NAcGIP[Lys(37)PAL] and Xenin-25[Lys(13)PAL] on reversal learning in wild-type mice. Reversal water maze training was initiated after 61 days treatment with saline, liraglutide, NAcGIP[Lys(37)PAL] and Xenin-25[Lys(13)PAL]. The acquisition phase involved 4 training sessions per day over 4 consecutive days. Illustrated is the average escape latency per training day (A), path length (B), and swim speed (C). Δp<0.05, ΔΔp<0.01 and ΔΔΔΔp<0.0001 vs. Wild-type Saline. Data represent mean ± SEM for 11-12 mice per group;

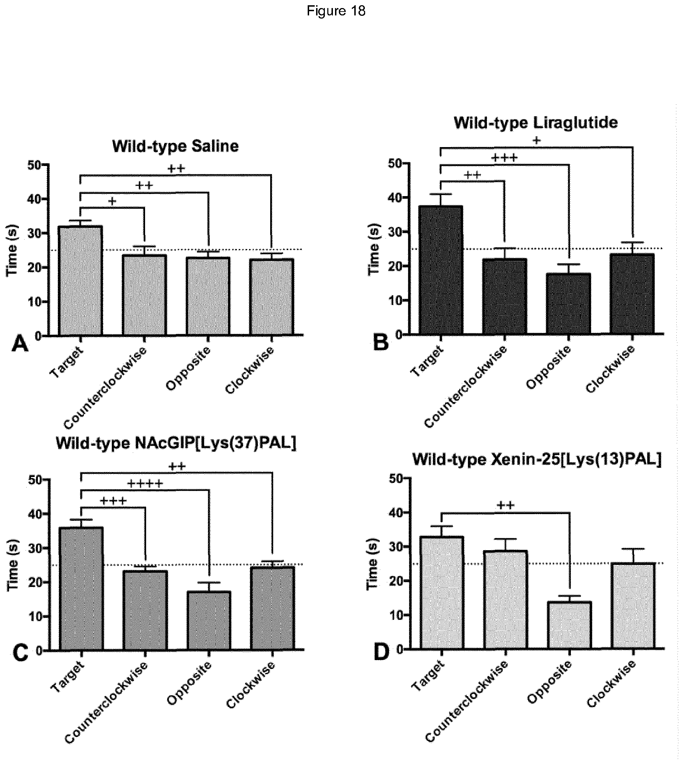

**Figure 18** is a graph illustrating the effects of chronic treatment with liraglutide, NAcGIP[Lys(37)PAL] and Xenin-25[Lys(13)PAL] on reversal memory in wild-type mice. A reversal probe trial was conducted 24 hours following the final reversal water maze training session as a measurement of reversal memory. Illustrated is time spent in each of the quadrants during the reversal probe trial by saline (A), liraglutide (B), NAcGIP[Lys(37)PAL] (C) and Xenin-25[Lys(13)PAL] (D) -treated wild-type mice, following 65 days treatment with each of the respective drugs. +p<0.05, ++p<0.01, +++p<0.001 and ++++p<0.0001 vs. target quadrant. Data represent mean ± SEM for 11-12 mice per group;

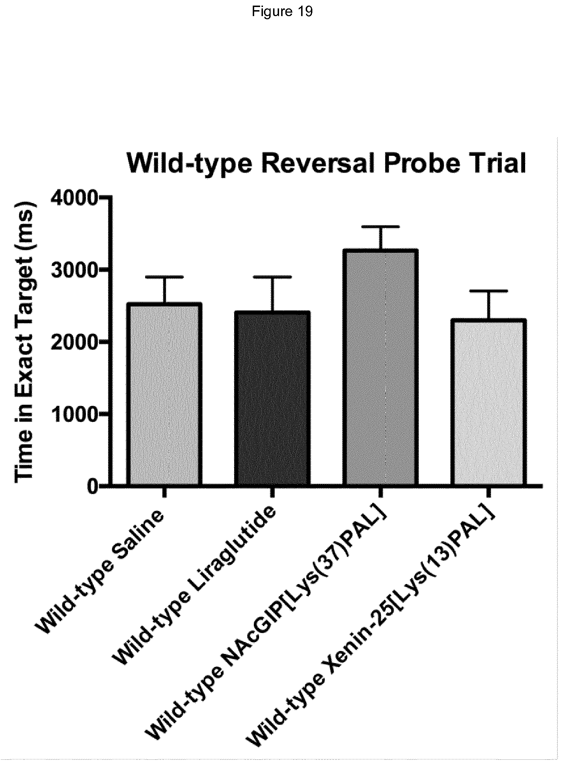

**Figure 19** is a graph illustrating the effects of chronic treatment with liraglutide, NAcGIP[Lys(37)PAL] and Xenin-25[Lys(13)PAL] on reversal spatial acuity in wild-type mice. Illustrated above is the amount of time spent within a 1 cm radius of the previous location of the escape platform during the probe trial, which took place 24 hours following the final reversal water maze training session, as an indication of spatial acuity in wild-type mice following 65 days treatment with respective drugs. Data represent mean ± SEM for 11-12 mice per group;

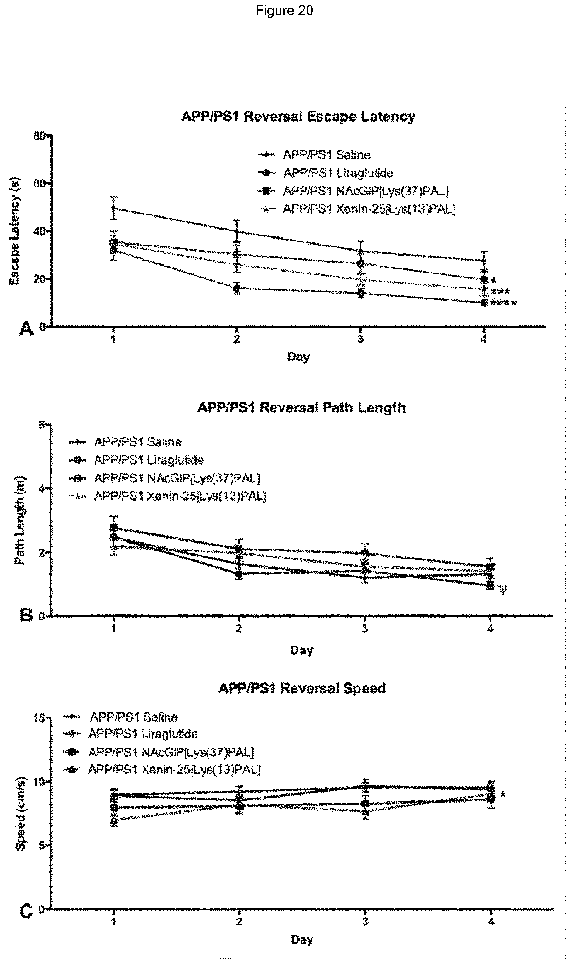

**Figure 20** is a graph illustrating the effects of chronic treatment with liraglutide, NAcGIP[Lys(37)PAL] and Xenin-25[Lys(13)PAL] on reversal learning in APP/PS1 mice. Reversal water maze training was initiated after 61 days treatment with saline, liraglutide, NAcGIP[Lys(37)PAL] and Xenin-25[Lys(13)PAL]. The acquisition phase involved 4 training sessions per day over 4 consecutive days. Illustrated is the average escape latency per training day (A), path length (B), and swim speed (C) of APP/PS1 mice. *p<0.05, ***p<0.001 and ****p<0.0001 vs. APP/PS1 Saline. ψp<0.05 vs. APP/PS1 NAcGIP[Lys(37)PAL]. Data represent mean ± SEM for 11-12 mice per group;

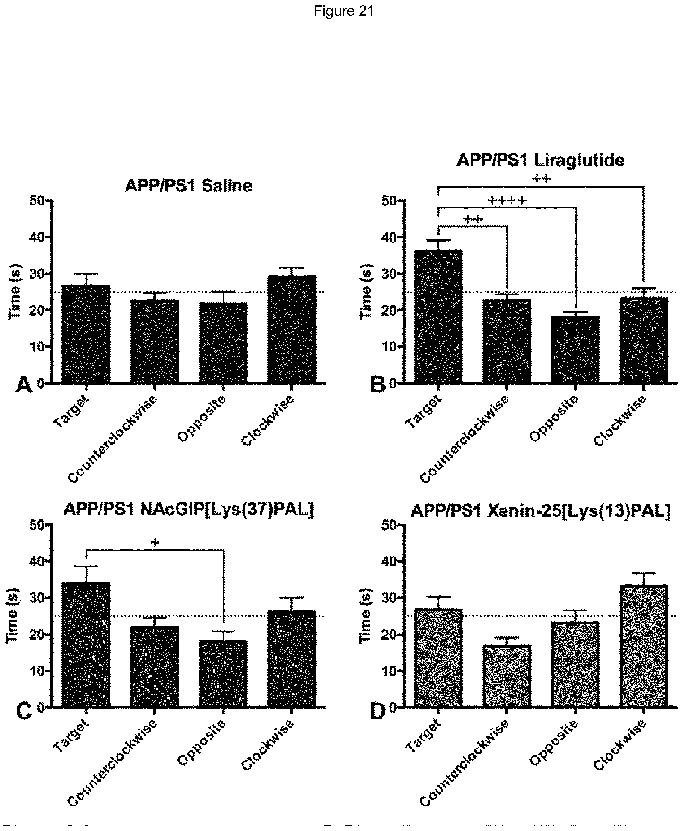

**Figure 21** is a graph illustrating the effects of chronic treatment with liraglutide, NAcGIP[Lys(37)PAL] and Xenin-25[Lys(13)PAL] on reversal memory in APP/PS1 mice. A reversal probe trial was conducted 24 hours following the final reversal water maze training session as a measurement of reversal memory. Illustrated is time spent in each of the quadrants during the reversal probe trial by saline (A), liraglutide (B), NAcGIP[Lys(37)PAL] (C) and Xenin-25[Lys(13)PAL] (D) -treated APP/PS1 mice, following 65 days treatment with each of the respective drugs. +p<0.05, ++p<0.01 and ++++p<0.0001 vs. target quadrant. Data represent mean ± SEM for 11-12 mice per group;

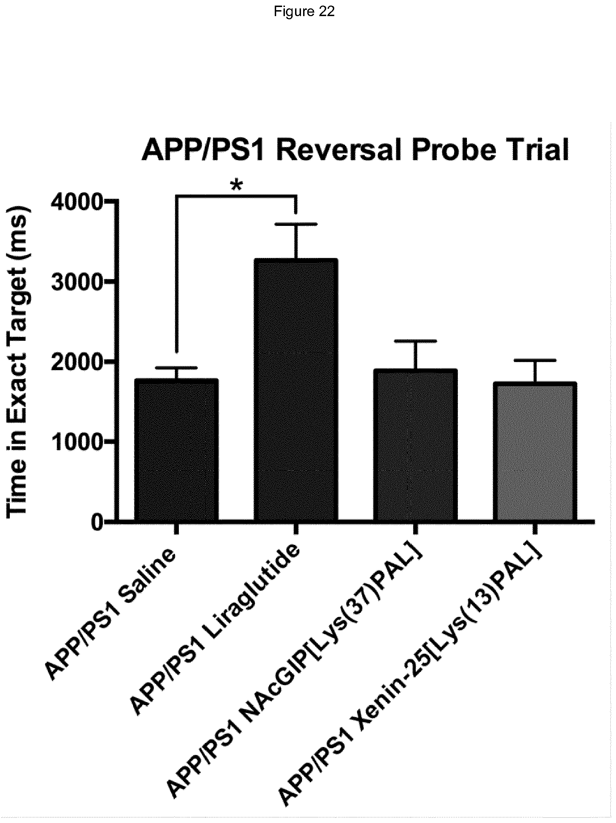

**Figure 22** is a graph illustrating spatial acuity of APP/PS1 liraglutide-treated mice is improved in a reversal water maze probe trial. Illustrated above is the amount of time spent within a 1 cm radius of the previous location of the escape platform during the probe trial, which took place 24 hours following the final reversal water maze training session, as an indication of spatial acuity in APP/PS1 mice following 65 days treatment with respective drugs. *p<0.05 vs. APP/PS1 Saline. Data represent mean ± SEM for 11-12 mice per group;

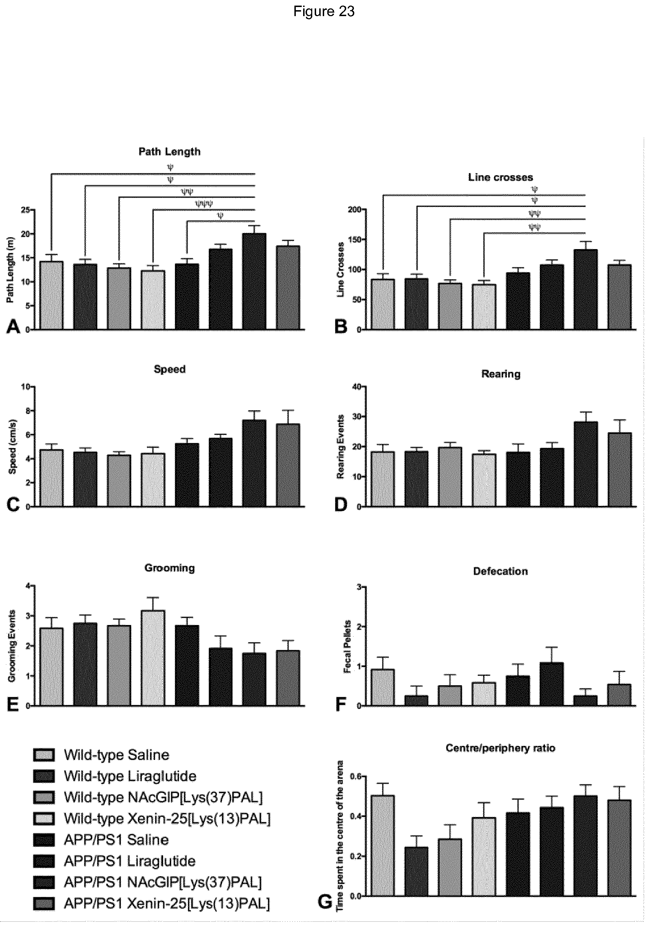

**Figure 23** is a graph illustrating the effects of chronic treatment with saline, liraglutide, NAcGIP[Lys(37)PAL] or Xenin-25[Lys(13)PAL] on open field behaviour in wild-type and APP/PS1 mice. In an open field task, path lengh (A), line crosses (B), speed (C), rearing (D), grooming (E), fecal pellets (F) and time spent in the centre of the arena (G) was measured after 66 days administration with each of the respective treatments in wild-type and APP/PS1 mice in order to assess the chronic effects of each treatment on spontaneous behaviour. ψp<0.05, ψψp<0.01 and ψψψp<0.001 vs. APP/PS1 NAcGIP[Lys(37)PAL]. Data represent mean ± SEM for 11-12 mice per group;

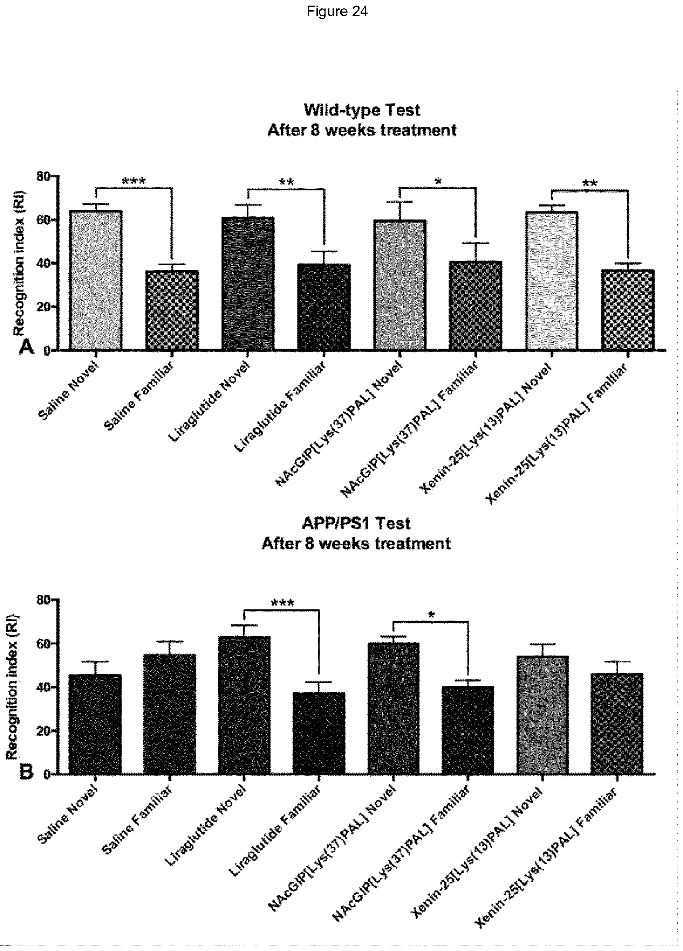

**Figure 24** is a graph illustrating the effects of chronic treatment with saline, liraglutide, NAcGIP[Lys(37)PAL] and Xenin-25[Lys(13)PAL] on recognition memory in wild-type and APP/PS1 mice. The test phase of the novel object recognition task took place 3 hours following an initial 10 minute acquisition phase. In the test phase, one of the identical objects from the acquisition phase was replaced with a novel object, not previously encountered. Illustrated are recognition indices for the novel object, compared with the familiar object in wild-type (A) and APP/PS1 (B) mice in the test phase following 67 days administration of respective treatments. *p<0.05, **p<0.01 and ***p<0.001 vs. novel object. Data represent mean ± SEM for 11-12 mice per group;

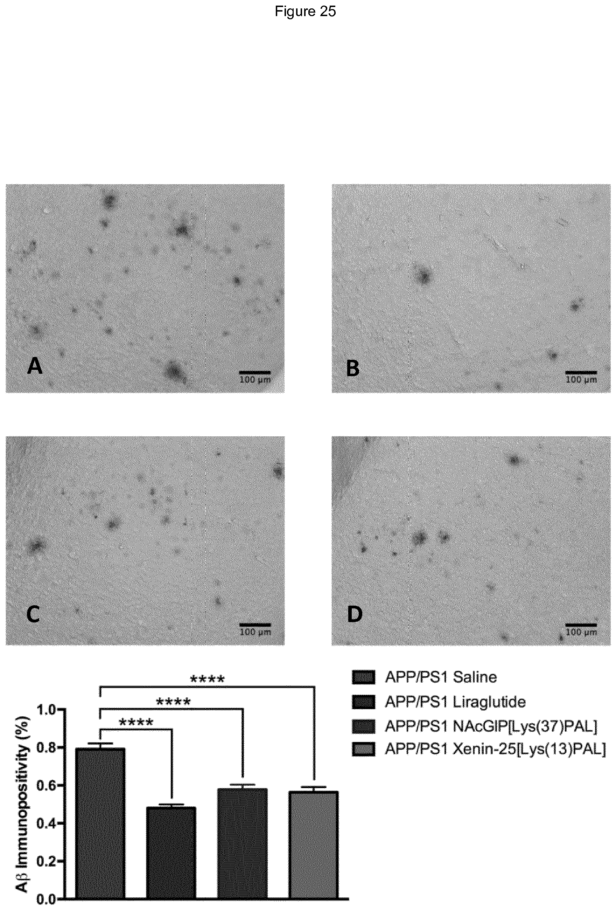

**Figure 25** is a graph illustrating Aβ plaque load in the brains of APP/PS1 mice treated with liraglutide, NAcGIP[Lys(37)PAL] and Xenin-25[Lys(13)PAL]. Representative images are shown that depict Aβ deposition in the cerebral cortex of saline (A), liraglutide (B), NAcGIP[Lys(37)PAL] (C) and Xenin-25[Lys(13)PAL] (D) -treated APP/PS1 mice following 10 weeks treatment with respective drugs. Also illustrated is overall quantification of Aβ immunopositivity in the cortex of each treatment group of APP/PS1 mice (E). ****p<0.0001 vs. APP/PS1 Saline. Data represent mean ± SEM for 6 mice per group;

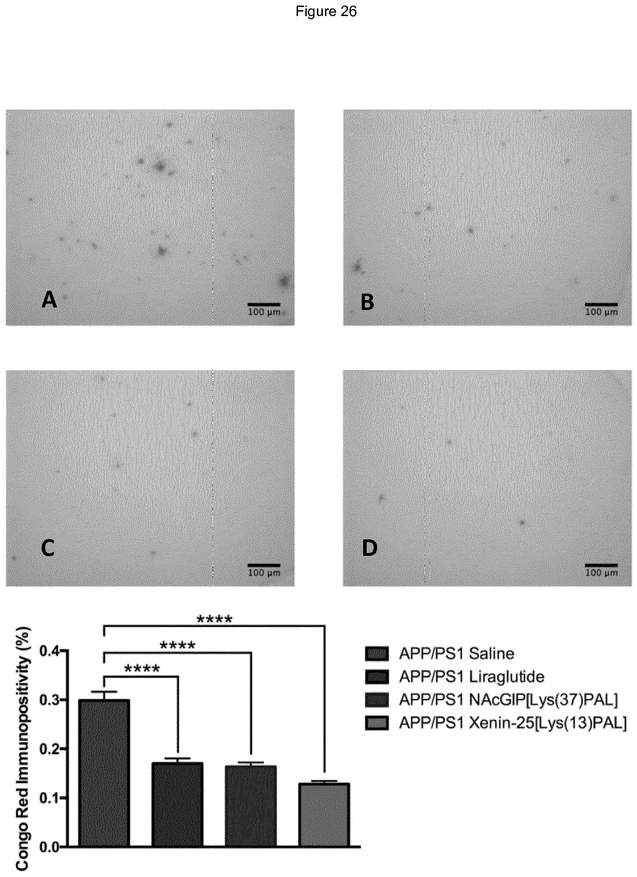

**Figure 26** is a graph illustrating dense-core congophilic plaque load in the brains of APP/PS1 mice treated with liraglutide, NAcGIP[Lys(37)PAL] and Xenin-25[Lys(13)PAL]. Representative images are shown that depict congo redpositive dense core Aβ plaques in the cerebral cortex of saline (A), liraglutide (B), NAcGIP[Lys(37)PAL] (C) and Xenin-25[Lys(13)PAL] (D) -treated APP/PS1 mice following 10 weeks treatment with respective drugs. Also illustrated is overall quantification of congophilic plaque load in the cortex of each treatment group of APP/PS1 mice (E). ****p<0.0001 vs. APP/PS1 Saline. Data represent mean ± SEM for 6 mice per group;

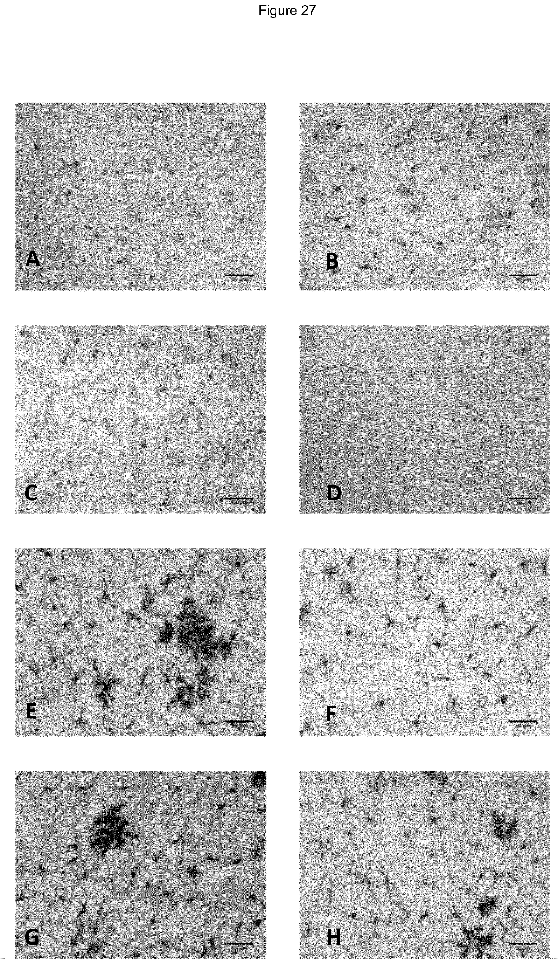

**Figure 27** is representative micrographs illustrating microglia in the cerebral cortex of drug-treated wild-type and APP/PS1 mice. Representative images are shown that depict Iba1 staining in the cortex of wild-type mice treated with saline (A), liraglutide (B), NAcGIP[Lys(37)PAL] (C) and Xenin-25[Lys(13)PAL] (D) and APP/PS1 mice that received saline (E), liraglutide (F) NAcGIP[Lys(37)PAL] (G) and Xenin-25[Lys(13)PAL] (H) once-daily for 10 weeks;

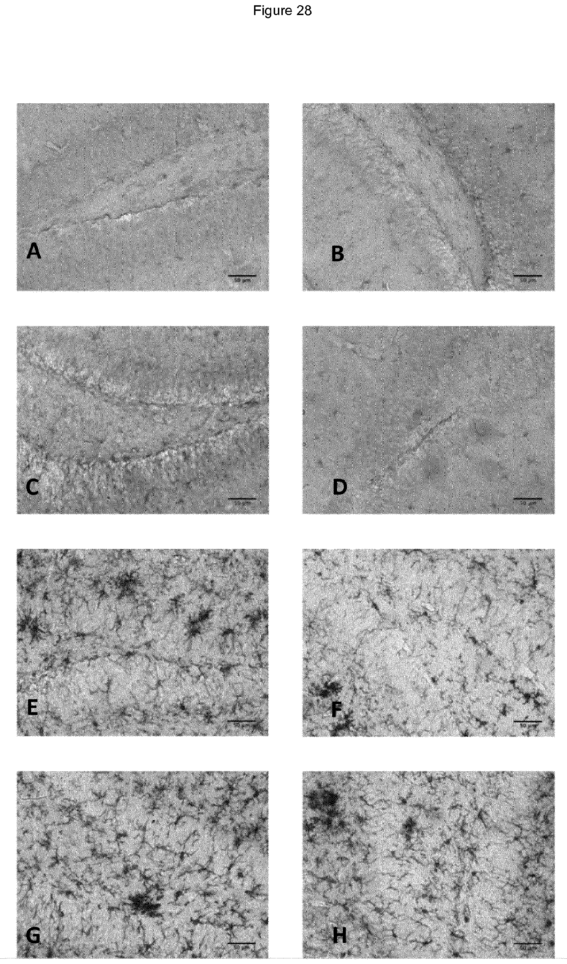

**Figure 28** is representative micrographs illustrating microglia in the dentate gyrus of drug-treated wild-type and APP/PS1 mice. Representative images are shown that depict Iba1 staining in the dentate gyrus of wild-type mice treated with saline (A), liraglutide (B), NAcGIP[Lys(37)PAL] (C) and Xenin-25[Lys(13)PAL] (D) and APP/PS1 mice that received saline (E), liraglutide (F) NAcGIP[Lys(37)PAL] (G) and Xenin-25[Lys(13)PAL] (H) once-daily for 10 weeks;

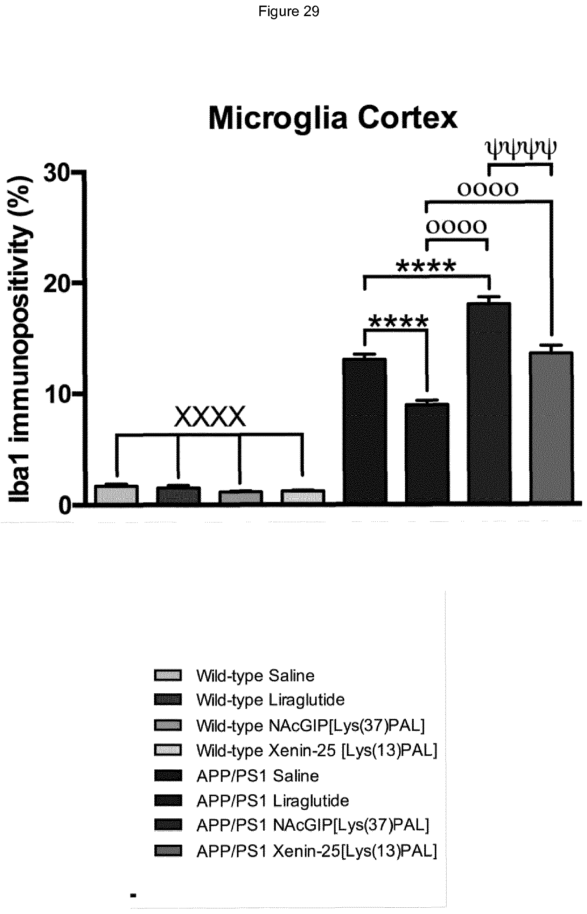

**Figure 29** is a graph illustrating microglial levels in the brains of wild-type and APP/PS1 mice treated with liraglutide, NAcGIP[Lys(37)PAL] and Xenin-25[Lys(13)PAL]. Illustrated above is overall quantification of Iba1 immunopositivity in the cerebral cortex (A) and dentate gyrus (B) of wild-type and APP/PS1 mice treated with saline, liraglutide, NAcGIP[Lys(37)PAL] and Xenin-25[Lys(13)PAL], oncedaily for 10 weeks. XXXXp<0.0001 vs. all APP/PS1 groups; **p<0.01, ****p<0.0001 vs. APP/PS1 Saline; op<0.05, oop<0.01, oooop<0.0001 vs. APP/PS1 Liraglutide ΨΨΨΨp<0.0001 vs. APP/PS1 NAcGIP[Lys(37)PAL]. Data represent mean ± SEM for 6 mice per group;

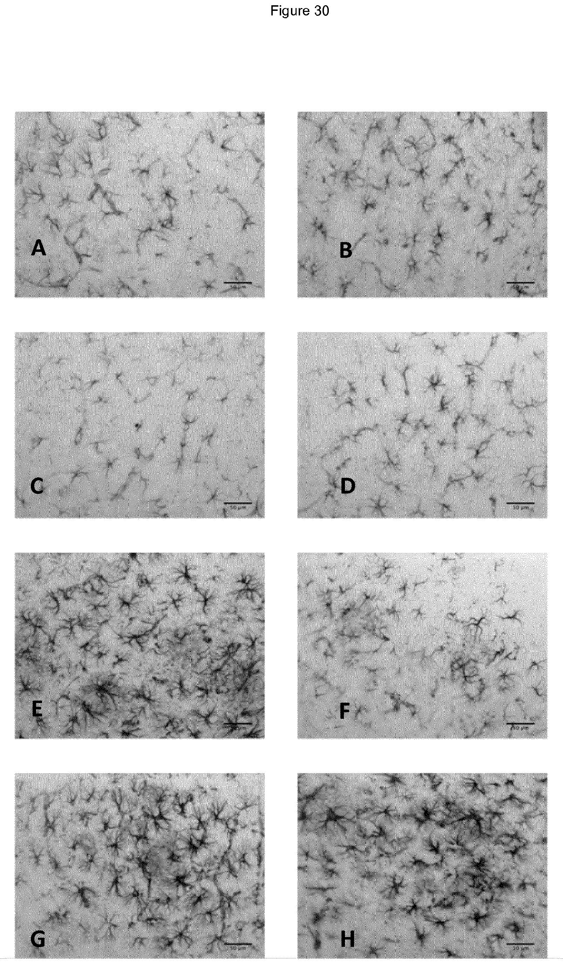

**Figure 30** is representative micrographs illustrating astrocytes in the cerebral cortex of drug-treated wild-type and APP/PS1 mice. Representative images are shown that depict GFAP staining in the cortex of wild-type mice treated with saline (A), liraglutide (B), NAcGIP[Lys(37)PAL] (C) and Xenin-25[Lys(13)PAL] (D) and APP/PS1 mice that received saline (E), liraglutide (F) NAcGIP[Lys(37)PAL] (G) and Xenin-25[Lys(13)PAL] (H) once-daily for 10 weeks;

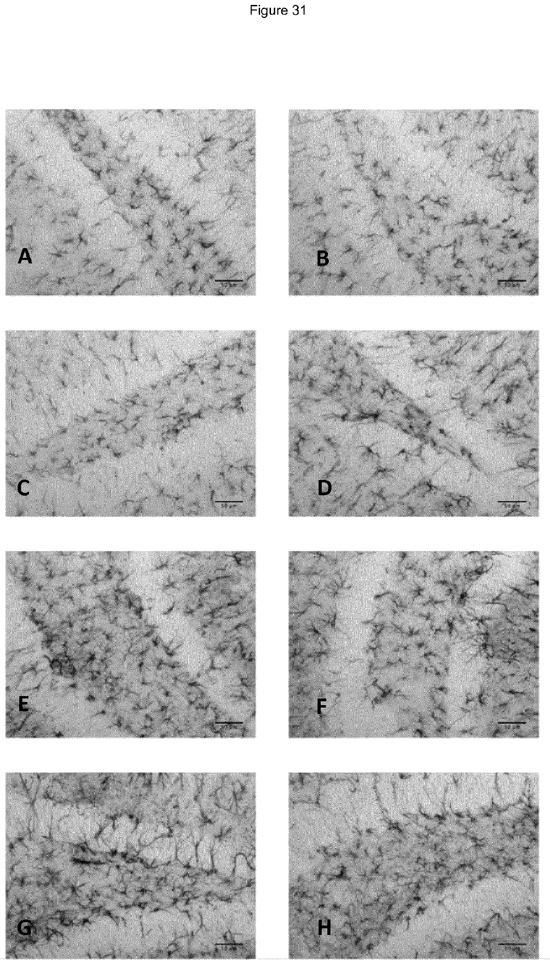

**Figure 31** is representative micrographs illustrating astrocytes in the dentate gyrus of drug-treated wild-type and APP/PS1 mice. Representative images are shown that depict GFAP staining in the dentate gyrus of wild-type mice treated with saline (A), liraglutide (B), NAcGIP[Lys(37)PAL] (C) and Xenin-25[Lys(13)PAL] (D) and APP/PS1 mice that received saline (E), liraglutide (F) NAcGIP[Lys(37)PAL] (G) and Xenin-25[Lys(13)PAL] (H) once-daily for 10 weeks;

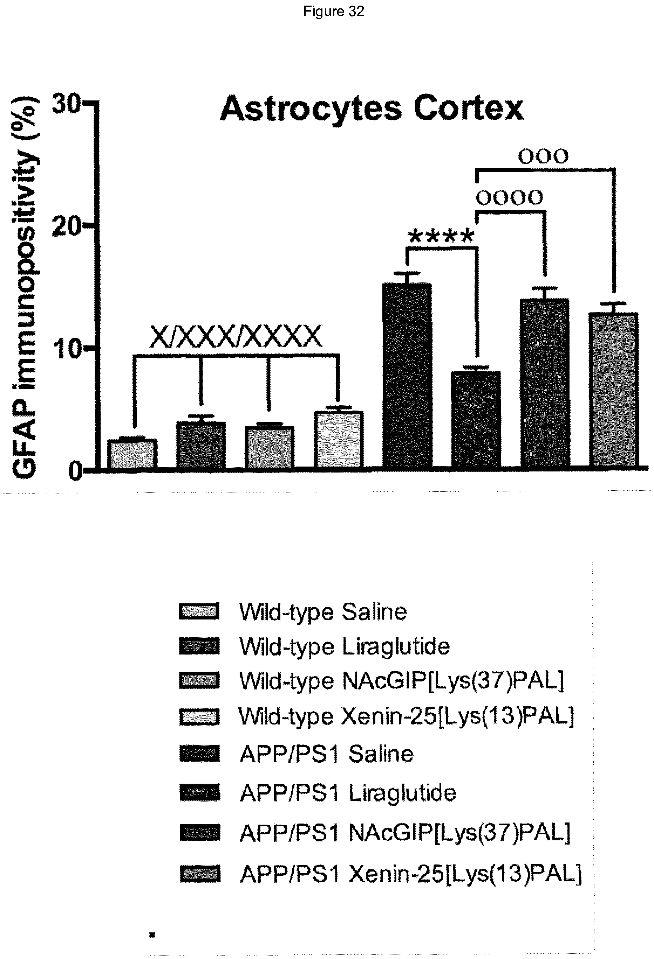

**Figure 32** is a graph illustrating astrocyte levels in the brains of wild-type and APP/PS1 mice treated with liraglutide, NAcGIP[Lys(37)PAL] and Xenin-25[Lys(13)PAL]. Illustrated above is overall quantification of GFAP immunopositivity in the cerebral cortex (A) and dentate gyrus (B) of wild-type and APP/PS1 mice treated with saline, liraglutide, NAcGIP[Lys(37)PAL] and Xenin-25[Lys(13)PAL], oncedaily for 10 weeks. X/XXX/XXXXp<0.05-p<0.0001 vs. all APP/PS1 groups; *p<0.05, ****p<0.0001 vs. APP/PS1 Saline; op<0.05 vs. APP/PS1 Liraglutide; +++p<0.001, ++++p<0.0001 vs. APP/PS1 Xenin-25[Lys(13)PAL]. Data represent mean ± SEM for 6 mice per group;

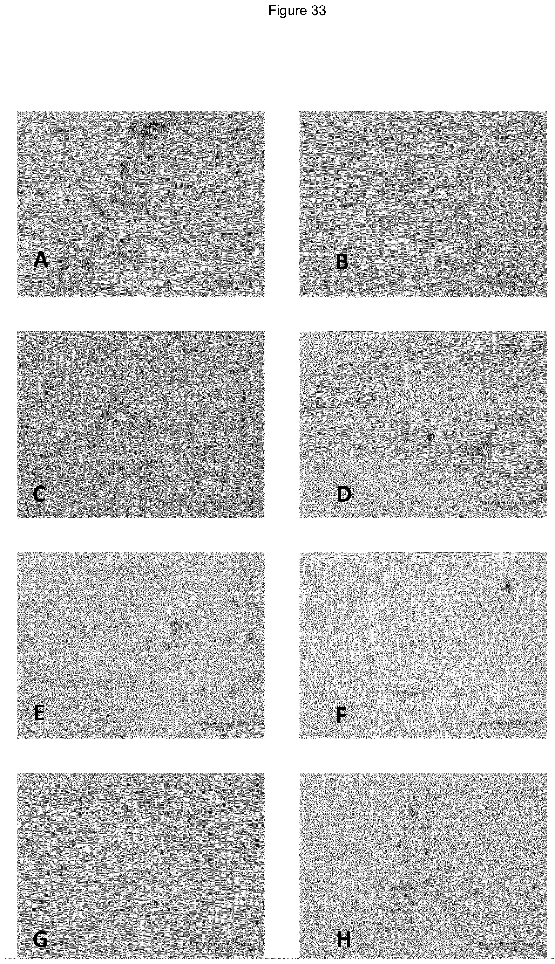

**Figure 33** depicts neurogenesis in the dentate gyrus of wild-type and APP/PS1 mice treated with liraglutide, NAcGIP[Lys(37)PAL] and Xenin-25[Lys(13)PAL]. Representative images above depict doublecortin staining in the dentate gyrus of wild-type mice treated with saline (A), liraglutide (B) NAcGIP[Lys(37)PAL] (C) and Xenin-25[Lys(13)PAL] (D) and APP/PS1 mice that received saline (E), liraglutide (F) NAcGIP[Lys(37)PAL] (G) and Xenin-25[Lys(13)PAL] (H) once-daily for 10 weeks;

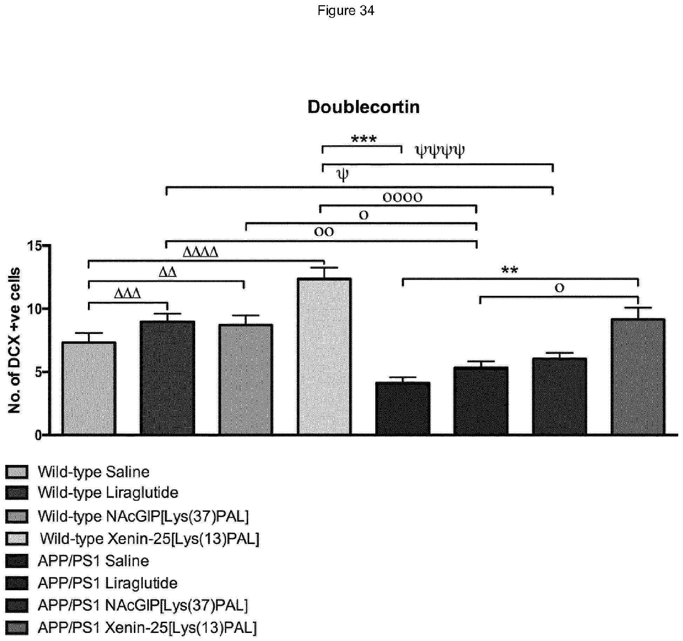

**Figure 34** is a graph illustrating Xenin-25[Lys(13)PAL] promotes neurogenesis in wild-type and APP/PS1 mice. Illustrated is quantification of doublecortin-positive cells in the dentate gyrus of wild-type and APP/PS1 mice treated with saline, liraglutide, NAcGIP[Lys(37)PAL] and Xenin-25[Lys(13)PAL] once-daily for 10 weeks. **p<0.01, ***p<0.001 and ****p<0.0001 vs. APP/PS1 Saline; ΔΔp<0.01, ΔΔΔp<0.001, ΔΔΔΔp<0.0001 vs. Wild-type Saline; op<0.05, oop<0.01 and oooop<0.0001 vs. APP/PS1 Liraglutide; ψp<0.05 and ΨΨΨΨρ<0.0001 vs. APP/PS1 NAcGIP[Lys(37)PAL]. Data represent mean ± SEM for 6 mice per treatment group;

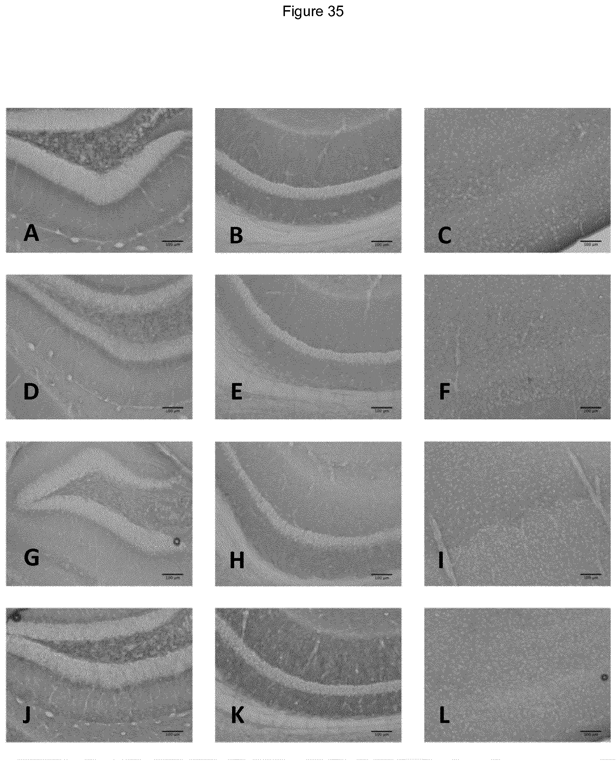

**Figure 35** is representative micrographs illustrating synapse density in wildtype mice. Illustrated are representative images of synaptophysin staining in the brains of wild-type mice treated with saline (A-C), liraglutide (D-F), NAcGIP[Lys(37)PAL] and Xenin-25[Lys(13)PAL] (J-L) once-daily for 10 weeks. A, D, G and J depict the polymorphic layer, granule cell layer and molecular layers of dentate gyrus. B, E, H and K depict stratum radiatum, stratum pyramidale and stratum oriens of the hippocampus and C, F, I and L depict the inner and outer layers of the cerebral cortex;

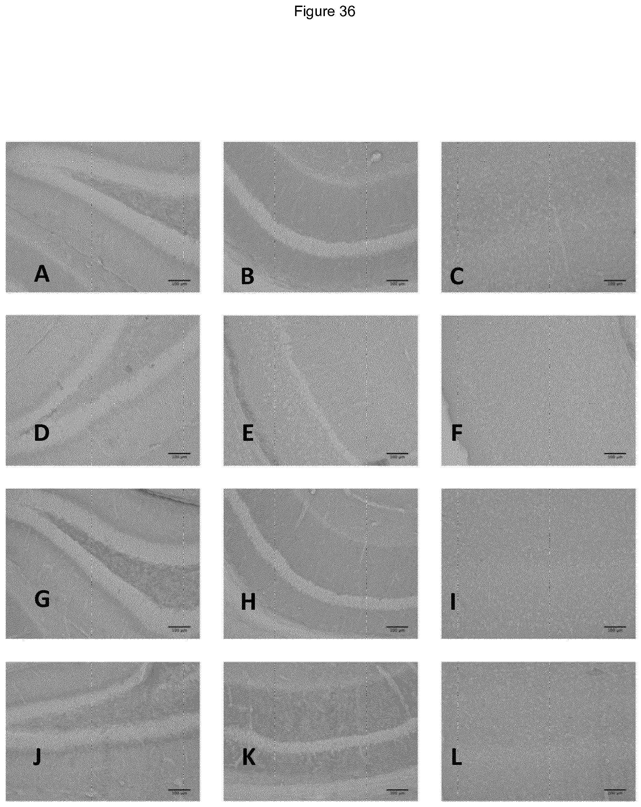

**Figure 36** is representative micrographs illustrating synapse density in APP/PS1 mice. Illustrated are representative images of synaptophysin staining in the brains of APP/PS1 mice treated with saline (A-C), liraglutide (D-F), NAcGIP[Lys(37)PAL] and Xenin-25[Lys(13)PAL] (J-L) once-daily for 10 weeks. A, D, G and J depict the polymorphic layer, granule cell layer and molecular layers of dentate gyrus. B, E, H and K depict stratum radiatum, stratum pyramidale and stratum oriens of the hippocampus and C, F, I and L depict the inner and outer layers of the cerebral cortex;

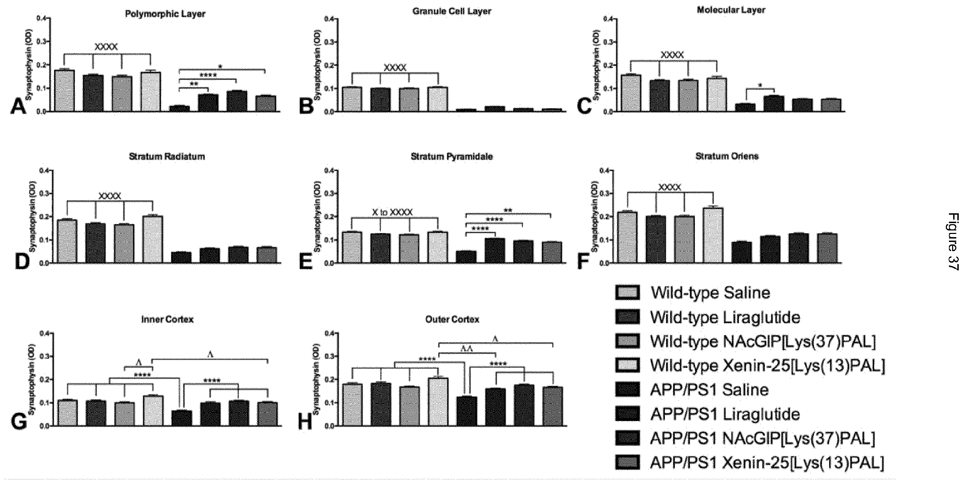

**Figure 37** is a graph illustrating synapse density in the brains of wild-type and APP/PS1 mice treated with liraglutide, NAcGIP[Lys(37)PAL] and Xenin-25[Lys(13)PAL]. Illustrated is overall quantification of synaptophysin optical density in the polymorphic layer (A), granule cell layer (B), and molecular layer of the dentate gyrus (C); stratum radiatum (D), stratum pyramidale (E) and stratum oriens (F) of the hippocampus and interior (G) and exterior (H) cerebral cortex. X/XXXXp<0.05-p<0.0001 vs. all groups of APP/PS1 mice, *p<0.05, **p<0.01, ***p<0.001 and ****p<0.0001 vs. APP/PS1 Saline, ∧p<0.05 and ∧∧p<0.01 vs. Wild-type Xenin-25[Lys(13)PAL]. Data represent mean ± SEM for 6 mice per group; and

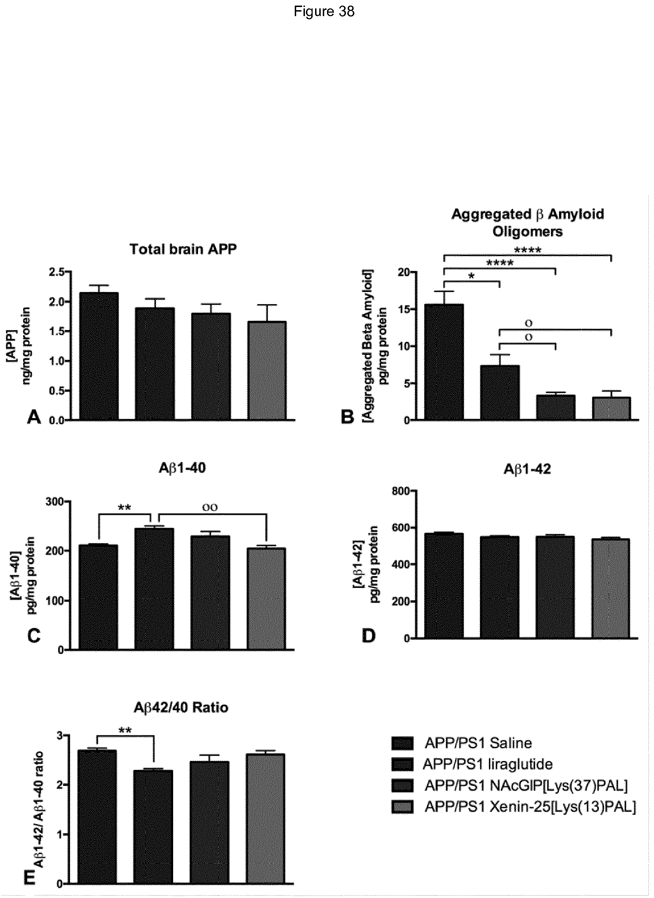

**Figure 38** is a graph illustrating soluble Aβ in the brains of APP/PS1 mice treated with liraglutide, NAcGIP[Lys(37)PAL], and Xenin-25[Lys(13)PAL] for 10 weeks. Illustrated is quantification, normalised to total protein content, of total brain APP (A), aggregated Aβ oligomers (B), Aβ1-40 (C), Aβ1-42 (D) and Aβ42/40 ratio (E) from brains of APP/PS1 mice treated with saline, liraglutide, NAcGIP[Lys(37)PAL] or Xenin-25[Lys(13)PAL] once-daily for 10 weeks. *p<0.05, **p<0.01 and ****p<0.0001 vs. APP/PS1 Saline; op<0.05 and oop<0.01 vs. APP/PS1 Liraglutide. Data represent mean ± SEM for 6 mice per treatment group.

### Examples

Embodiments of the present invention will now be described by way of non-limiting examples.

### Materials & Methods

### Animals

APP*swe*/PS1ΔE9 mice with a C57BI/6 background were bred at the animal unit of Ulster University. Heterozygous males were bred with wild-type C57/BI6 females bought locally (Harlan, UK). Offspring were ear punched and genotyped using PCR with primers specific for the APP sequence (Forward "GAATTCCGACATGACTCAGG", Reverse: "GTTCTGCTGCATCTTGGACA"). Mice not expressing the transgene were used as wild-type controls. Male animals were used in all studies. Animals were caged individually and maintained on a 12/12 light-dark cycle (lights on at 08h00, off at 20h00), in a temperature-controlled room (T:21.5°C±1). Food and water were available *ad libitum.* Animals were handled daily for two weeks prior to commencement of the study. APP/PS1 and wild-type animals were 7 months of age when treatment began. They were randomized and were administered their designated treatment, liraglutide (2.5 nm/kg bw), N-AcGIP(Lys³⁷PAL37), xenin25(Lys¹³PAL) or saline (0.9% w/v), intraperitoneally (i.p.) once daily (at 15:00h). Treatment groups comprised n =12-14. All experiments were licensed by the UK home office in accordance with the Animal (scientific procedures) Act of 1986.

### Peptides

N-AcGIP(Lys³⁷PAL), xenin25(Lys¹³PAL) and liraglutide were purchased from EZ Biolabs (Indiana, USA). The purity of peptides was analysed by reversed-phase HPLC and characterised using matrix assisted laser desorption/ionisation time of flight (MALDI-TOF) mass spectrometry, with a purity > 99%. Peptides were reconstituted in ultrapure® water to a concentration of 1 mg/ml in polypropylene tubes and frozen in aliquots to permit fresh preparation of doses required for injection.

### Experimental design and treatments

APP/PS1 and wild-type animals were 7 months of age when treatment began. They were randomised and assigned to 4 treatment groups per genotype, such that there were 8 groups in total. Wild-type and APP/PS1 mice were respectively divided into four groups that received either liraglutide (2.5 nmol/kg bw), NAcGIP[Lys(37)PAL] (2.5 nmol/kg bw) Xenin-25[Lys(13)PAL] (25 nmol/kg bw) or saline (0.9% w/v), i.p. once daily (at 15h00) for 8-10 weeks. Treatment groups comprised n=12-14. Liraglutide, NAcGIP[Lys(37)PAL] and Xenin-25[Lys(13)PAL] were purchased from EZ Biolabs (Indiana, USA). The purity of peptides was analysed by reversed-phase HPLC and characterised using matrix assisted laser desorption/ionization - time of flight (MALDI-TOF) mass spectrometry, with a purity > 99%. Peptides were reconstituted in ultrapure® water to a concentration of 1 mg/ml in polypropylene tubes and frozen in aliquots to permit fresh preparation of doses required for injection. Peptides were then prepared in 0.9% NaCl prior to administration in a volume of 10 ml/kg.

### Open field task

Mice were subjected to the open field task (OFT) in order to assess anxiety and locomotor activity. In short, mice were placed into a circular arena (58 cm in diameter and 31 cm in height) and allowed to explore freely for 5 minutes. The arena was illuminated by a 60W light positioned 2 m above the arena, directly adjacent to the position of the camera. Mice movements were recorded using a computerised tracking system (Biosignals, New York, USA), with which OFT performance was also analysed. Grooming events, rearing events and defecation were recorded manually to indicate levels of anxiety. Grooming was defined as behaviour in which the mouse stopped running and started licking, chewing or scratching its fur. Rearing was defined as behaviour in which the mouse stopped running and lifted both of its front paws off the floor and reared up on its hind legs. Defecation was measured by counting the number of fecal pellets in the arena after each trial. Path length, speed and linearity were recorded digitally as an indication of locomotor activity. The OFT was conducted 24 hours prior to the novel object recognition task, in order to acclimatize the mice to the apparatus and diminish the natural stress and anxiety that an unfamiliar environment might cause. The arena was thoroughly cleaned with ethanol (70%) between each animal to prevent build up of olfactory cues.

### Novel Object Recognition Task

After 9 days (group 1), 25 days (group 2), 8 weeks (group 3) or 19 weeks (group 4) mice were assessed in the novel object recognition task (ORT). This task exploits the natural preference for novelty of rodents and is used as a measure of working memory in mice. In short, mice were placed into the arena for an initial habituation phase, along with two identical objects; either two red cubes (1.8 cm wide) or two white balls (2 cm wide) which were secured to the floor of the arena using Blu tack, 15 cm from the wall. The arena was dimly lit with a 60 W light bulb, positioned 2m above the arena. Mice were allowed to freely explore the arena, with the familiar objects for 10 minutes. Their movements were recorded with an overhead camera and the time spent exploring the two familiar objects was calculated using Tracker software (Biosignals, New York, USA) within a 2 cm radius of the objects. The mice were then removed and returned to their home cage for 3 hours. After the interval, mice were then placed back into the arena for the test phase, along with one familiar object from the habituation phase and one novel object (either a red cube or a white ball). The familiar object that was used and the position of the novel object (left or right) were randomised to reduce the influence of any confounding factors. Previous studies show that neither object nor place preference is apparent with the use of the red cube and white ball. Again, mice were allowed to freely explore for 10 minutes and their movements recorded. The arena was cleaned with 70% ethanol between each trial to remove potentially confounding olfactory cues. Data are expressed as a recognition index (RI), which is calculated for each object in both the acquisition and test phases. RI is defined as the amount of time spent exploring object A or B over the total time spent exploring both objects multiplied by one hundred (tA or tB/(tA + tB) x 100).

### Morris Water Maze

After 10 days (group 1), 26 days (group 2), 8 weeks (group 3) or 19 weeks (group 4) mice were assessed in the Morris water maze (MWM). The maze was 40cm in height, with a diameter of 120 cm and was filled with water maintained at 25°C ± 1°C. A small escape platform (10 x 6.5 x 21.5 cm) was placed on the centre of one quadrant, 25 cm from the edge and hidden 1 cm below the surface of the water. Visual cues were placed on the walls of the room. The acquisition training phase of the MWM consisted of four 90 second trials per day, for 4 consecutive days. Mice were placed into a water-filled tank and were allowed to swim freely. The point at which the each mouse was placed into pool was randomised across the trials. Using extra-maze visual cues mice had to learn the location of an escape platform, which was hidden 1 cm below the surface of the water in the centre of the south-west quadrant of the pool. If a mouse failed to find the platform within 90 seconds, it was guided there, and encouraged to remain there for 20 seconds. Mice were then returned to their home cage for an inter-trial period of 20 minutes. Twenty-four hours following the final training day mice were placed back into the water, with the escape platform removed for a single probe trial. Mice were allowed to swim freely for 90 seconds and the time spent in the quadrant that previously contained the escape platform was measured as an indication of functional working memory. Time spent in the exact location of the escape platform was recorded as a measure of spatial acuity.

### Reversal Water Maze

After 15 days (group 1), 31 days (group 2), 9.5 weeks (group 3) or 20.5 weeks (group 4) mice were subjected to the reversal water maze (RWM), in which the escape platform was moved from the south-west to the north-west quadrant. This modification is designed to assess cognitive flexibility, or the ability to relearn the new location of the platform. There were 4 trials per day, for 3/4 consecutive days, followed by a final reversal probe trial.

### Surgery and LTP recording in the hippocampus area CA1

Mice were used for *in vivo* electrophysiological experiments in order to assess long-term potentiation. Mice were anesthetized with urethane (ethyl carbamate, 1.8g/kg ip.) for the duration of all experiments. The skull was exposed and 3 holes with 0.8mm diameter were drilled. Electrodes (tungsten with Teflon coating, Bilaney, Kent, United Kingdom) were implanted, 1.5mm posterior and 1.0mm lateral to the midline for the recording electrode, and 2.0mm posterior to bregma and 1.5mm lateral to the midline for the stimulating electrode. The electrodes were slowly lowered through the cortex and the upper layers of the hippocampus and into the CA1 region until the appearance of a negative deflecting excitatory postsynaptic potential (EPSP) that had a latency of ca 10msec. Recordings of field excitatory postsynaptic potentials (fEPSPs) were made from the stratum radiatum in the CA1 region of the right hippocampal hemisphere in response to stimulation of the Schaffer collateral/commissural pathway. fEPSPs were recorded on a computerized stimulating and recording unit (PowerLab, ADI Instruments) in which the trigger threshold was adjustable. The triggered unit activated a constant current stimulus isolation unit (Neurolog, United Kingdom). The data acquisition system was triggered simultaneously to record all events. Sampling speed was at 20kHz for recordings of fEPSPs. In order to assess the excitability and determine an appropriate voltage for high frequency stimulation and recording, the response of each of the mouse to increasing voltage was measured in an input/output curve at intervals of 0.25 V from 2.0 V to a maximum of 5 V (or until a maximal response was elicited). Stimulation voltage was then adjusted to generate approximately 60% of the maximum fEPSP, and the recording of the baseline was measured at this value. The high frequency stimulation protocol for inducing LTP consisted of 4 trains of 100 stimuli with an interstimulus interval of 5msec (200Hz) and an inter-train interval of 2 sec. This LTP induction protocol was chosen to prevent saturation of LTP and thus allow the possibility to detect facilitation of LTP. LTP was measured as a percentage of baseline fEPSP slope recorded over a 15-minute period before application of high frequency stimulation. This value was taken as 100% of the excitatory postsynaptic potential slope and all recorded values were normalized to this baseline value. The post high frequency stimulation LTP data were then analysed by a two-way repeated measures ANOVA with treatment group as the between subject factor and time as the within subject factor.

### Histology

Animals were perfused trans-cardially with PBS buffer followed by ice-cold 4% paraformaldehyde in PBS. Brains were removed and hemispheres separated. One hemisphere was fixed in 4% paraformaldehyde (PFA) until processing and the other was snap frozen in liquid nitrogen and stored at -80°C for future analysis. Hemi-brains were then transferred from PFA to 30% sucrose for at least 24 hours to prevent the formation of ice crystals upon freezing. Brains were then set with freezing spray (Envirotech Freezing Spray, Thermoscientific, UK; Cat. No. 6769038) and left in holders within the cryostat (Leica Microsystems, Milton Keynes, UK), which was set at -25°C. Brains were then cut in 40 µm coronal sections at a depth of -2 to -3 Bregma using a Leica cryostat, and collection began on appearance of the hippocampus (-0.94 µm to bregma) until approximately 3.28 µm to bregma. For stereological purposes the first section to be kept was selected at random, and placed in a 24 well plate, and one section in every 7 were collected sequentially, such that 7 sets of sections, with 10-13 sections each were collected from each mouse brain. Plates were stored at -20°C until required.

Sections were chosen according to stereological rules with the first section taken at random and every 6th section afterwards. Staining was carried out for beta-amyloid plaques, and for congophilic dense-core amyloid plaques. Staining was also carried out for synaptophysin, in order to determine synapse numbers, and Doublecortin (DCX), a marker for immature neurons, as a measure of neurogenesis. Staining was carried out for GFAP; a marker of astrocytes, Iba1; a marker of microglia, 8-oxoguanine; a marker of oxidative stress, IRS-1 pSer616 and synaptophysin, which was used to determine synapse density. All sections were incubated in 0.08%-5% H2O2 to quench endogenous peroxidase activity and permeabilised using 0.01%-0.03% Triton X.

The Iba1 protocol required incubation of sections in 0.05 M trisodium citrate (pH 9) at 90°C for 30 minutes to enhance antigen recognition. Non-specific binding was then blocked using 5%-10% normal serum prior to incubation with mouse, anti-GFAP (1:250, in PBS with 0.3% Triton X; Merck Millipore, Feltham, UK; Cat. No. MAB3402) polyclonal, rabbit anti-Iba1 (1:200 in PBS with 2% goat serum and 0.3% Triton X; Wako Chemicals, Neuss, Germany; Cat. No. 019-19791), polyclonal, rabbit anti-Aβ antibody (1:200 in TBS; Invitrogen, Paisley, UK; Cat. No. 71-5800), goat antimouse doublecortin (1:200 in PBS with 2% rabbit serum; Santa Cruz Biotechnology Inc. Dallas, Texas, USA; Cat. No. Sc-8066) or synaptophysin (1:200; Abcam, Cambridge, UK; Cat. No. ab7837) overnight at 4°C. After blocking the sections in 5% normal serum to avoid non-specific antibody binding, they were incubated with rabbit polyclonal anti amyloid beta peptide (1:250, Invitrogen, UK, 71-5800) or goat polyclonal anti doublecortin (1:200, Santacruz, USA, sc-710), or rabbit anti-synaptophysin (1:200, Abcam, Cambridge, MA, 7837-500). After overnight incubation at 4°C, the sections were incubated in respective secondary antibodies. The following day, sections were incubated with respective secondary antibodies and visualisation was achieved using Vectastain Elite and SG substrate (Vectamount AQ, Vector Laboratories Ltd, Peterborough, UK; Cat. No. H5501). Dense-core amyloid plaques were detected using Congo red. Sections were washed with PBS and were then mounted onto salinised slides and allowed to air dry, immersed in distilled water for 30 seconds and then a solution of saturated sodium chloride (NaCl) for 20 minutes. Prior to the addition of the slides, sodium hydroxide (NaOH) was added to the saturated NaCl. Slides were then transferred from the NaCl solution to 0.2% alkalised Congo red (Sigma Aldrich, St Louis, Mo, USA; Cat. No. 60910) diluted in saturated NaCl solution for 30 minutes. Slides were then dehydrated in graded ethanol and immersed in xylene 3 times for 5 minutes, before cover slips were mounted using Vectamount permanent mounting medium (Vector laboratories, Burlingame, CA; Cat. No. H-5000). Images were obtained of stained sections using a Zeiss Axio microscope (Axio scope A1, Zeiss, Germany) and the percentage area stained in each image (2 images per section, approximately 8-10 sections per mouse, 16-20 images total) was quantified using a multi threshold plug-in within Image J (ImageJ, U. S. National Institutes of Health, Bethesda, Maryland, USA). Analysis of percentage stained area was conducted for all markers, except synaptophysin, for which analysis of OD was performed. Images were converted to grayscale, a threshold was set and the average percentage area positively stained was calculated for each brain. All immunohistochemistry analysis was conducted in a blinded manner, whereby codes were applied to microscope slides by colleagues not involved in the studies. Codes were only broken upon completion of analysis.

### Human APP ELISA brain extraction

Cell extraction buffer (Invitrogen, Camarillo, CA, USA; Cat. No. FNN0011) composed of 10 mM Tris, pH 7.4, 100 mM NaCl, 1 mM EDTA, 1 mM EGTA, 1 mM NaF, 20 mM Na4P2O7, 2 mM Na3VO4, 1% Triton X, 10% glycerol, 0.1% SDS, 0.5% deoxycholate, was supplemented with 250 µl protease inhibitor cocktail per 5 ml (Sigma-Aldrich, St Louis, Mo, USA; Cat. No. P8340) immediately prior to use. A volume of 4 ml of cell extraction buffer was added per 100 mg brain tissue that had been homogenised under liquid nitrogen. Samples were lysed for 30 minutes on ice and vortexed at 10 minute intervals, before being centrifuged at 13,000 rpm for 10 minutes at 4°C. The resulting supernatant was aliquoted into a new eppendorf and kept on ice for immediate analysis of APP levels.

### Human APP ELISA brain extraction

Cell extraction buffer (Invitrogen, Camarillo, CA, USA; Cat. No. FNN0011) composed of 10 mM Tris, pH 7.4, 100 mM NaCl, 1 mM EDTA, 1 mM EGTA, 1 mM NaF, 20 mM Na4P2O7, 2 mM Na3VO4, 1% Triton X, 10% glycerol, 0.1% SDS, 0.5% deoxycholate, was supplemented with 250 µl protease inhibitor cocktail per 5 ml (Sigma-Aldrich, St Louis, Mo, USA; Cat. No. P8340) immediately prior to use. A volume of 4 ml of cell extraction buffer was added per 100 mg brain tissue that had been homogenised under liquid nitrogen. Samples were lysed for 30 minutes on ice and vortexed at 10 minute intervals, before being centrifuged at 13,000 rpm for 10 minutes at 4°C. The resulting supernatant was aliquoted into a new eppendorf and kept on ice for immediate analysis of APP levels.

### Aggregated Aβ Oligomer Sandwich ELISA

Each test sample was diluted 1:10 in Standard Diluent Buffer provided by the Aggregated Aβ ELISA Kit (Invitrogen, Camarillo, CA, USA; Cat. No. KHB3491). Aggregated Aβ standards were prepared in duplicate from Human Aggregated Aβ Standard solution (Invitrogen, Camarillo, CA, USA; Cat. No. KHB3491) from 5.7 ng/ml to 0.0195 ng/ml, diluted in Standard Diluent Buffer. Standard and test samples (100 µl) were pipetted into wells coated with monoclonal antibody specific for the N-terminus of Aβ using a 96-well plate (Invitrogen, Camarillo, CA, USA; Cat. No. KHB3491). The first 2 wells were left empty, as chromogen blanks while 100 µl standard diluent buffer was added to zero wells. Wells were covered with an adhesive plate cover and samples were incubated for 2 hours at room temperature. Using a multi wash III tricontinent machine (Tricontinent, Grass Valley, CA, USA), wells were washed 4 times with wash buffer (Invitrogen, Camarillo, CA, USA; Cat. No. KHB3491) diluted 1:25 in distilled water beforehand. After washing, 100 µl of biotinylated detection antibody (Invitrogen, Camarillo, CA, USA; Cat. No. KHB3491) was added to each well except chromogen blank wells and incubated for 1 hour at room temperature. After 4 washes in wash buffer, wells were incubated for 30 minutes at room temperature with 100 µl of horseradish peroxidase-labeled streptavidin (SAV-HRP) (Invitrogen, Camarillo, CA, USA; Cat. No. KHB3491) except chromogen blank wells. Excess SAV-HRP was removed and wells were washed 4 times with wash buffer. Each well was then incubated with 100 µl of stabilised chromogen (Invitrogen, Camarillo, CA, USA; Cat. No. KHB3491) for 20-30 minutes at room temperature in the dark until a blue colour developed. Finally, 100 µl of stop solution (Invitrogen, Camarillo, CA, USA; Cat. No. KHB3491) was added, eliciting a blue to yellow colour change in the solution. The plate was introduced to a spectrophotometer (Flexstation 3 Benchtop multimode microplate reader, Molecular Devices Inc, Sunnyvale, CA, USA) immediately after adding stop solution. Absorbance of each well was performed at 450 nm having blanked the plate reader against a chromogen blank. Absorbance readings of the standards were plotted against the standard concentration and Human Aggregated Aβ standard curve generated was used to determinate aggregated Aβ concentrations in each sample. Aggregated Aβ concentrations of each test sample were then divided by the corresponding brain protein concentrations, as determined by the Bradford assay.

### Aβ1-40 and Aβ1-42 ELISA brain extraction

Brain tissue, previously homogenised under liquid nitrogen was mixed with 8 volumes of ice-cold guanidine buffer (5.0 M guanidine HCI (Sigma-Aldrich, St Louis, Mo, USA; Cat. No. G3272)/50 mM Tris HCI (Sigma-Aldrich, St Louis, Mo, USA; Cat. No. T5941, pH 8.0) at 4°C overnight. The homogenates were then further diluted (1:10) with ice-cold reaction buffer BSAT-DPBS (Dulbecco's PBS (Sigma-Aldrich, St Louis, Mo, USA; Cat. No. D1408) with 5% BSA and 0.03% Tween 20 (Sigma-Aldrich, St Louis, Mo, USA; Cat. No. P9416), supplemented with 250 µl protease inhibitor cocktail per 5 ml (Sigma-Aldrich, St Louis, Mo, USA; Cat. No. P8340). Samples were then centrifuged for 20 minutes at 4°C. The supernatant was transferred into a new eppendorf and stored on ice until use.

### Aβ 1-40 Sandwich ELISA

Each test sample was diluted 1:3 in Standard Diluent Buffer provided by the Aβ1-40 ELISA Kit (Invitrogen, Camarillo, CA, USA; Cat. No. KHB3481). Aβ1-40 standards were prepared in duplicate from Human Aβ1-40 Standard solution (Invitrogen, Camarillo, CA, USA; Cat. No. KHB3481) from 0.5 ng/ml to 0.00781 ng/ml, diluted in Standard Diluent Buffer. Standard and test samples (50 µl) were pipetted into wells coated with monoclonal antibody specific for the N-terminus of Aβ1-40 using a 96-well plate (Invitrogen, Camarillo, CA, USA; Cat. No. KHB3481). The first 2 wells were left empty, as chromogen blanks, while 50 µl standard diluent buffer was added to zero wells. A rabbit detection antibody (50 µl) that recognised the C-terminus of the 1-40 Aβ sequence was then added to each well, except for the chromogen blanks. Wells were then covered with an adhesive plate cover and samples were incubated for 3 hours at room temperature with shaking. Using a multi wash III tricontinent machine (Tricontinent, Grass Valley, CA, USA), wells were washed 4 times with wash buffer (Invitrogen, Camarillo, CA, USA; Cat. No. KHB3481) diluted 1:25 in distilled water beforehand. After washing, 100 µl of anti-rabbit IgG HRP was added to each well, except the chromogen blanks. The wells were covered with a plate cover and the samples were incubated for 30 minutes at room temperature. Excess anti-rabbit IgG HRP was removed and wells were washed 4 times with wash buffer. Each well was then incubated with 100 µl of stabilised chromogen (Invitrogen, Camarillo, CA, USA; Cat. No. KHB3481) for 20-30 minutes at room temperature in the dark until a blue colour developed. Finally, 100 µl of stop solution (Invitrogen, Camarillo, CA, USA; Cat. No. KHB3481) was added, eliciting a blue to yellow colour change in the solution. The plate was introduced to a spectrophotometer (Flexstation 3 Benchtop multimode microplate reader, Molecular Devices Inc, Sunnyvale, CA, USA) immediately after adding stop solution. Absorbance of each well was read at 450 nm having blanked the plate reader against a chromogen blank. Absorbance readings of the standards were plotted against the standard concentration and Human Aβ1-40 standard curve generated was used to determinate Aβ1-40 concentrations in each sample. Aβ1-40 concentrations of each test sample were then divided by the corresponding brain protein concentrations, as determined by the Bradford assay.

### Aβ 1-42 Sandwich ELISA

Each test sample was diluted 1:3 in Standard Diluent Buffer provided by the Aβ1-42 ELISA Kit (Invitrogen, Camarillo, CA, USA; Cat. No. KHB3441). Aβ1-42 standards were prepared in duplicate from Human Aβ1-42 Standard solution (Invitrogen, Camarillo, CA, USA; Cat. No. KHB3441) from 1 ng/ml to 0.01563 ng/ml, diluted in Standard Diluent Buffer. Standard and test samples (50 µl) were pipetted into wells coated with monoclonal antibody specific for the N-terminus of Aβ using a 96-well plate (Invitrogen, Camarillo, CA, USA; Cat. No. KHB3441). The first 2 wells were left empty, as chromogen blanks while 50 µl standard diluent buffer was added to zero wells. A rabbit detection antibody (50 µl) that recognised the C-terminus of the 1-42 Aβ sequence was then added to each well, except for the chromogen blanks. Wells were then covered with an adhesive plate cover and samples were incubated for 3 hours at room temperature with shaking. Using a multi wash III tricontinent machine (Tricontinent, Grass Valley, CA, USA), wells were washed 4 times with wash buffer (Invitrogen, Camarillo, CA, USA; Cat. No. KHB3441) diluted 1:25 in distilled water beforehand. After washing, 100 µl of anti-rabbit IgG HRP was added to each well, except the chromogen blanks. The wells were covered with a plate cover and the samples were incubated for 30 minutes at room temperature. Excess anti-rabbit IgG HRP was removed and wells were washed 4 times with wash buffer. Each well was then incubated with 100 µl of stabilised chromogen (Invitrogen, Camarillo, CA, USA; Cat. No. KHB3441) for 20-30 minutes at room temperature in the dark until a blue colour developed. Finally, 100 µl of stop solution (Invitrogen, Camarillo, CA, USA; Cat. No. KHB3441) was added, eliciting a blue to yellow colour change in the solution. The plate was introduced to a spectrophotometer (Flexstation 3 Benchtop multimode microplate reader, Molecular Devices Inc, Sunnyvale, CA, USA) immediately after adding stop solution. Absorbance of each well was performed at 450 nm having blanked the plate reader against a chromogen blank. Absorbance readings of the standards were plotted against the standard concentration and Human Aβ1-42 standard curve generated was used to determinate Aβ1-42 concentrations in each sample. Aβ1-42 concentrations of each test sample were then divided by the corresponding brain protein concentrations, as determined by the Bradford assay.

### Statistics

Data were analysed using the program Prism (Graphpad software Inc., USA), with the level of probability set at 95%. Results are expressed as means ± SEM. Data were analyzed by 1-way or 2-way ANOVA, followed by post hoc tests, or student t-tests.

### Example 1

*In Vivo* studies establishing effects of peptide analogues of the invention on early cognitive decline in APP/PS1 mice.

APP/PS1 mice were injected with either saline, liraglutide, or peptide analogue of the invention (Xenin-25[Lys(13)PAL]) once daily for 10 weeks. Xenin-25[Lys(13)PAL] and liraglutide were assessed for their ability to preserve levels of synaptophysin (a presynaptic vesicle protein), a marker of synaptic density. Synaptic loss is associated with cognitive decline. Therefore, the ability to preserve synapses is indicative of neuroprotective properties, with increased synaptic integrity associated with improved cognitive function.

As illustrated in Figure 1 (A-F), quantification of levels of expression of synaptophysin demonstrate that all groups of wild-type mice had significantly higher expression levels in the hippocampus than APP/PS1 mice treated with saline, liraglutide, or Xenin-25[Lys(13)PAL]. Synaptophysin staining in all groups of wild-type mice was significantly greater than APP/PS1 saline-treated mice in the interior (G), and exterior (H) cortex. Synaptophysin staining levels in liraglutide and Xenin-25[Lys(13)PAL] treated APP/PS1 mice were equivalent to all groups of wild-type mice in the interior cortex (G), while they were significantly reduced in liraglutide (p<0.01) and Xenin-25[Lys(13)PAL] treated APP/PS1 groups compared with Xenin-25[Lys(13)PAL]-treated wild-type mice in the exterior cortex (H).

Liraglutide-treated APP/PS1 animals had significantly higher synaptophysin levels than saline-treated APP/PS1 mice in the polymorphic layer (A, p<0.001), the molecular layer (C, p<0.05), the stratum pyramidale (E, p<0.0001) and the interior (G, p<0.0001) and exterior (H, p<0.0001) cortex. Xenin-25[Lys(13)PAL] treated APP/PS1 mice had significantly higher synaptophysin levels than saline-treated APP/PS1 mice in the polymorphic layer (A, p<0.01), the stratum pyramidale (E, p<0.01) the interior (G, p<0.0001) and exterior (H, p<0.0001) cortex, indicating the protective ability of both liraglutide and Xenin-25[Lys(13)PAL] on synapse numbers. Synaptic loss is associated with the progression of Alzheimer's disease.

These data show both Xenin-25[Lys(13)PAL] and liraglutide to preserve synapses in the hippocampus and cortex (both of which are important brain regions in the context of learning and memory), further demonstrating their use in treating neurological disorders.

### Reference Example 2

Analysis of synaptic density following treatment with peptide analogues of the invention in APP/PS1 mice

APP/PS1 mice were injected with saline, liraglutide and NAcGIP[Lys(37)PAL]once daily for 10 weeks; and NAcGIP[Lys(37)PAL]and liraglutide were assessed for their ability to preserve levels of synaptophysin.

As illustrated in Figure 2, while synaptophysin levels were observed to be comparable in wild-type mice irrespective of treatment, synaptic density is partially preserved with both liraglutide and NAcGIP[Lys(37)PAL]treatment in APP/PS1 mice, suggesting that treatment with both drugs may have an impact on preventing synaptic loss.

Specifically, NAcGIP[Lys(37)PAL]treated APP/PS1 mice had significantly higher expression levels than saline-treated APP/PS1 mice in the polymorphic layer (A, p<0.0001), the stratum pyramidale (E, p<0.0001), the stratum oriens (F, p<0.05) and the interior (G, p<0.0001) and exterior (H, p<0.0001) cortex, indicating the protective ability of NAcGIP[Lys(37)PAL]on synapse numbers. The effects were comparable to those observed in liraglutide treated APP/PS1 mice. Preservation of synapses in the hippocampus and cortex, which are important brain regions in the context of learning and memory, by NAcGIP[Lys(37)PAL]and liraglutide further demonstrate use in treating neurological disorders.

### Example 3

### Effects of peptide analogues of the invention on key biomarkers of AD

Studies were conducted in the APP/PS1 mouse model to assess the chronic effect of Xenin-25[Lys(13)PAL] in comparison to liraglutide and saline controls, on beta amyloid and dense core congophilic plaque load as well as neurogenesis and inflammation. Histological markers were analysed following 10 weeks of treatment.

As illustrated in Figure 3 (A-D), beta amyloid plaque load was reduced by 30% in Xenin-25[Lys(13)PAL] treated mice compared with saline treated controls (p<0.0001). In line with the reduction in beta amyloid, APP/PS1 mice treated with Xenin-25[Lys(13)PAL] displayed a highly significant reduction (55%; p<0.0001) in dense core congophilic plaque load compared with saline treated controls. This Xenin-25[Lys(13)PAL]-induced reduction in dense core plaques was significantly greater than the 36% reduction induced by liraglutide treatment (p<0.001; see Figure 3 E-H). Such a finding suggests that peptide analogues of the invention outperform liraglutide in its ability to reduce formation of dense core plaques, which are closely associated with neuronal cell loss and advanced Alzheimer's disease pathology.

When assessed for its effects on inflammation, IBA-1 levels of Xenin-25[Lys(13)PAL] were comparable to saline-treated APP/PS1 mice (see Figure 3 I-L).

In addition to its effect on beta amyloid and dense core plaque load, Xenin-25[Lys(13)PAL] demonstrated an ability to significantly increase neurogenesis in APP/PS1 mice (Figure 3 M-P). Briefly, APP/PS1 mice treated with Xenin-25[Lys(13)PAL] had 122% more (p<0.0001) doublecortin positive neurons than saline-treated APP/PS1 mice, and significantly more than liraglutide treated APP/PS1 mice (p<0.01), indicating that peptide analogues of the invention stimulate neurogenesis more effectively than liraglutide in the APP/PS1 model of Alzheimer's disease.

### Reference Example 4

### Effects of peptide analogues of the invention on key biomarkers of AD

Studies were conducted in the APP/PS1 mouse model to assess the effect of NAcGIP[Lys(37)PAL] in comparison to liraglutide and saline controls, on beta amyloid and dense core congophilic plaque load, inflammation and neurogenesis.

As illustrated in Figure 4 (A-D), beta amyloid plaque load was significantly reduced by NAcGIP[Lys(37)PAL] (27% reduction) when compared with saline treated controls (p<0.0001). In line with the reduction in beta amyloid plaque load, APP/PS1 mice treated with NAcGIP[Lys(37)PAL] also displayed a significant reduction (45%) in dense core congophilic plaque load (p<0.0001; Figure 4 EH). No significant difference in beta amyloid plaque load was observed between liraglutide and NAcGIP[Lys(37)PAL] treated mice.

When assessed for its effects on inflammation, IBA-1 levels of NAcGIP(Lys³⁷PAL)-treated mice were comparable to saline-treated APP/PS1 mice (see Figure 4 I-L).

In addition, NAcGIP[Lys(37)PAL] demonstrated an ability to partially restore neurogenesis in treated APP/PS1 mice (Figure 4 M-P). APP/PS1 mice treated with NAcGIP[Lys(37)PAL] had significantly more (+46%) doublecortin positive neurons than saline treated APP/PS1 mice (p<0.05). There were no significant differences between liraglutide and saline treated APP/PS1 mice, and NAcGIP[Lys(37)PAL]-treated mice. In a t-test comparing each of the drug-treated groups with saline treated APP/PS1 mice, both liraglutide (p<0.05) and NAcGIP[Lys(37)PAL]-treated mice (p<0.01), had significantly more doublecortin neurons than their untreated APP/PS1 control littermates suggesting both drugs increase neurogenesis.

### Example 5

### Peptide analogues of the invention reduce soluble oligomer levels

As illustrated in Figure 5, Xenin-25[Lys(13)PAL] and liraglutide did not altar total APP (5A) or Aβ1-42 levels (5D) in APP/PS1 mice. Liraglutide treatment was associated with increased Aβ1-40 levels (5C) and a significantly reduced Aβ42/40 ratio (5E) compared with saline and Xenin-25[Lys(13)PAL] treated APP/PS1 mice.

Xenin-25[Lys(13)PAL]-treatment resulted in a highly significant 80% reduction in aggregated beta amyloid oligomers compared with saline treated APP/PS1 mice (5B, p<0.001). This was greater than the reduction of 54% observed in liraglutde-treated mice compared with controls (5B, p<0.01). Such a significant reduction would be expected to improve neuronal communication *in vivo.*

### Reference Example 6

### Peptide analogues of the invention reduce soluble oligomer levels

As illustrated in Figure 6, NAcGIP[Lys(37)PAL] and liraglutide did not significantly altar total APP (6A) or Aβ1-40 (6C) or Aβ1-42 levels (6D) in APP/PS1 mice. Liraglutide treatment was associated with a significantly reduced Aβ42/40 ratio (6E) compared with saline and NAcGIP[Lys(37)PAL]-treated APP/PS1 mice. NAcGIP[Lys(37)PAL] treatment resulted in a highly significant 78% reduction in aggregated beta amyloid oligomers compared with saline treated APP/PS1 mice (6B, p<0.001). This was greater than the reduction of 54% observed in liraglutide-treated mice compared with controls (6B, p<0.01). The reduction observed may account for the improved performance in the object recognition task as beta amyloid oligomers are reported to have detrimental effects on learning and memory (see Example 7 and Figure 7).

### Example 7

### Dose Response Study

Male C57BI/6 mice were bred at Ulster University and aged to 8-10 weeks before commencement of treatment. Animals were caged individually and maintained on a 12/12 light-dark cycle (lights on at 08h00, off at 20h00), in temperature-controlled room (T:21.5°C±1). Food and water were available ad libitum. Mice were injected with liraglutide, N-AcGIP[Lys(37)PAL], or Xenin-25[Lys(13)PAL] at 3 doses; 0.25nmol/kg, 2.5nmol/kg, and 25nmol/kg. Another group were injected with saline as a control. Animals were injected with their respective treatment once daily (at 15:00) for 21 days before being subjected to in vivo LTP. All experiments were licensed by the UK home office in accordance with the Animal (scientific procedures) Act of 1986.

When testing the inducibility of LTP in the hippocampus in vivo, using a weak stimulation protocol, saline-treated wild-type mice induced weak LTP (see Figures 7 A-C).

On analysis of post high frequency stimulation baselines liraglutide treatment was associated with enhanced LTP at lower concentrations (Figure 7A). In a 2-way ANOVA there was no significant effect of time (p>0.999). However, peptide analogue administration at 0.25nmol/kg was associated with an increased induction of LTP compared with saline treated mice (P=0.0068). At 2.5nmol/kg there was no effect over time (p>0.999), however there was a highly significant increase in LTP associated with peptide analogue administration (p<0.0001). At 25nmol/kg there was no significant effect of time (p>0.999). However, peptide analogue treatment significantly increased inducibility of LTP (p=0.001).

On analysis of post high frequency stimulation baselines NAcGIP[Lys(37)PAL] treatment was associated with enhanced LTP at higher concentrations (Figure 7B). In a 2-way ANOVA there was no significant effect of time (p=0.824). However, administration of peptide analogues of the present invention at 0.25nmol/kg was associated with reduced induction of LTP compared with saline treated mice (P<0.0001). At 2.5nmol/kg and 25nmol/kg there was no significant effect of time (p>0.999). However, peptide analogue treatment significantly increased inducibility of LTP at both concentrations (P=0.0001), with a marginally superior effect observed at the 2.5nmol/kg dose.

On analysis of post high frequency stimulation baselines Xenin-25[Lys(13)PAL treatment was associated with enhanced LTP at higher concentrations (Figure 7C). In a 2-way ANOVA there was no significant effect of time (p>0.999) or peptide analogue at 0.25nmol/kg (p=0.395), compared with saline treated mice. At 2.5nmol/kg there was no significant effect of time (p>0.999). However, treatment with peptide analogues of the present invention significantly increased inducibility of LTP (P=0.0008), while treatment with the 25nmol/kg dose was associated with significant effect over time (p=0.0055), and peptide analogue (p<0.0001).

### Example 8

Acute effects of treatment on locomotor function and anxiety.

Wild-type and APP/PS1 mice were injected with saline, liraglutide, NAcGIP[Lys(37)PAL] or Xenin-25[Lys(13)PAL] 30 minutes prior to being introduced to the open field arena in order to assess acute effects of drugs on spontaneous locomotor behaviour and anxiety. Path length (Figure 8A), line crosses (Figure 8B), and speed (Figure 8C) were, for the most part, similar between treated and untreated groups, with the exception of the APP/PS1 Xenin-25[Lys(13)PAL]-treated mice who displayed significantly increased path length, line crosses and speed compared with wild-type saline-treated mice (p<0.05). APP/PS1 Xenin-25[Lys(13)PAL]-treated mice also made significantly more line crosses than APP/PS1 liraglutide-treated mice (p<0.05). Rearing behaviour (Figure 8D) of APP/PS1-liraglutide-treated mice was significantly reduced compared with all but the wild-type liraglutide-treated group (p<0.05-p<0.0001). Additionally, rearing behaviour in the wild-type liraglutide-treated mice was significantly reduced compared with the APP/PS1 saline-treated group (p<0.05). Number of grooming events (Figure 8E), and fecal pellets produced in 5 minutes (Figure 8F) were not significantly different across genotype or treatment groups. However, time spent in the centre of the arena was significantly reduced in APP/PS1 liraglutide-treated mice, compared with the APP/PS1 saline-treated mice (p<0.05) suggestive of an increase in anxiety levels after acute liraglutide administration in the APP/PS1 model (Figure 8G).

### Example 9

### Acute effects of treatment on recognition memory

As illustrated in Figure 9, no place preference was apparent in either wild-type (A) or APP/PS1 mice (B). Place preference is calculated based on the time spent exploring identical objects, during the acquisition phase of the object recognition task. Assessment of place preference allows us to exclude that preference for objects is affected by their location within the apparatus. Object recognition memory in response to acute administration of saline, liraglutide, NAcGIP[Lys(37)PAL] and Xenin-25[Lys(13)PAL] is illustrated in Figure 10. All groups of wild-type mice (Figure 10A) displayed intact recognition memory and preferentially explored the novel over familiar object (p<0.05-p<0.0001). In contrast, none of the APP/PS1 groups (Figure 10B) could discriminate between novel and familiar objects, indicating that impaired recognition memory is apparent at the point of initiation of treatment in the APP/PS1 model.

### Example 10

### Chronic effects of treatment on spatial learning and memory

Morris water maze training began after 8 weeks treatment. Animals continued to receive treatment throughout behavioural testing. Training began at 09:00 daily, and drugs were administered at 15:00 to exclude the potential for acute drug effects on performance. During the acquisition phase of the Morris water maze task, animals completed 4 trials per day over 4 consecutive days. Average daily performance of wild-type mice is illustrated in Figure 11. Two-way repeated measures ANOVA demonstrated that there was no significant difference in the acquisition phase in wild-type mice treated with saline, liraglutide, NAcGIP[Lys(37)PAL] or Xenin-25[Lys(13)PAL]. Escape latency (Figure 11A) significantly decreased over time, as expected (p<0.0001), however drug had no impact on the escape latencies observed (p=0.4463). In line with escape latency, path length (Figure 11B) was not significantly different between treatment groups of wild-type mice, (p=0.24), however path length decreased over time (p<0.0001). Speed (Figure 11C) was not significantly altered by drug treatment (p=0.71), however speed was variable over time (p=0.0022).

Spatial memory was assessed in a probe trial conducted on day 5, 24 hours following the final training session (Figure 12). In wild-type saline-treated mice, a one-way ANOVA indicated that there was a significant difference between quadrants (p<0.0001). Dunnett's multiple comparisons post-hoc test demonstrated that saline-treated wild-type mice spent significantly more time in the target quadrant than the counterclockwise (CCW; p<0.001), opposite (OPP; p<0.0001) and clockwise (CW; p<0.05) quadrants, indicating intact spatial memory. Liraglutide-treated wild-type mice demonstrated a significant difference between quadrants (p=0.0018). Post-hoc analyses demonstrated that liraglutide-treated mice spent significantly more time in the target quadrant than the CCW (p<0.05), OPP (p<0.01) and CW (p<0.01) quadrants. NAcGIP[Lys(37)PAL]-treated wild-type mice demonstrated a significant difference between quadrants (p=0.0008). Dunnett's multiple comparisons posthoc test demonstrated that NAcGIP[Lys(37)PAL]-treated wild-type mice spent significantly more time in the target quadrant than the CCW (p<0.01), OPP (p<0.05) and the CW (p<0.001) quadrants. Xenin-25[Lys(13)PAL]-treated wildtype mice also demonstrated a significant difference between quadrants (p=0.0005). Dunnett's post-hoc test demonstrated that Xenin-25[Lys(13)PAL]-treated wild-type mice spent significantly more time in the target quadrant than the CCW (p<0.05), OPP (p<0.001) and CW (p<0.01) quadrants. Together this demonstrates that spatial memory of wild-type mice, in the Morris water maze, is unaffected by chronic treatment with liraglutide, NAcGIP[Lys(37)PAL] or Xenin-25[Lys(13)PAL].

Spatial acuity was assessed by measuring the amount of time spent swimming at the precise location where the escape platform had been located during training (Figure 13). No significant differences were observed between wild-type treatment groups.

Average daily performance of APP/PS1 mice in the acquisition phase of the Morris water maze task is illustrated in Figure 14. An overall two-way repeated measures ANOVA demonstrated that escape latency (Figure 14A) in APP/PS1 mice was significantly impacted by drug treatment (p=0.0280), and decreased significantly over time (p<0.0001). Tukey's multiple comparisons test highlighted an overall difference in escape latency between the APP/PS1 saline and liraglutide-treated groups (p<0.05), with no differences observed in NAcGIP[Lys(37)PAL] orXenin-25[Lys(13)PAL]-treated APP/PS1 mice. An overall repeated measures two-way ANOVA demonstrated path length was significantly impacted by drug (p=0.0252) and time (p<0.0001). Tukey's post-hoc multiple comparisons test demonstrated that path length was significantly reduced in APP/PS1 liraglutide-treated mice compared with APP/PS1 Xenin-25[Lys(13)PAL]-treated mice overall (Figure 14B; p<0.05). On analysis of swim speed, an overall repeated measures two-way ANOVA demonstrated that there was a significant impact of drug treatment (p=0.0277) and time (p=0.0008) in APP/PS1 mice. Tukey's multiple comparisons test demonstrated an overall difference in the swimming speed of APP/PS1 Xenin-25[Lys(13)PAL]-treated mice compared with APP/PS1 saline-treated mice (p<0.05). Analysis on a day by day basis demonstrated that Xenin-25[Lys(13)PAL]-treated mice swam significantly slower than APP/PS1 saline treated mice on day 3, and that APP/PS1 NAcGIP[Lys(37)PAL]-treated mice swam significantly slower on day 4 compared with APP/PS1 saline-treated mice (Figure 14C; p<0.01).

Spatial memory of APP/PS1 mice was assessed in a probe trial conducted on day 5, 24 hours following the final training session. The escape platform was removed and spatial memory was assessed using the percentage of time spent swimming in the target quadrant (Figure 15). In APP/PS1 mice treated with saline (Figure 15A; p=0.2934), NAcGIP[Lys(37)PAL] (Figure 15C; p=0.4470) and Xenin-25[Lys(13)PAL] (Figure 15D; p=0.1492), one-way ANOVA indicated that there was no statistically significant difference between time spent in each quadrant during the probe trial, indicating an impairment of spatial memory. Liraglutide-treated APP/PS1 mice demonstrated a significant difference between time spent in each of the quadrants (p=0.0036) during the probe trial. Dunnett's post hoc analysis demonstrated that liraglutide-treated APP/PS1 mice spent significantly more time in the target quadrant than the CCW (p<0.05), OPP (p<0.01) and CW (p<0.05) quadrants, indicating that spatial memory was restored with chronic liraglutide treatment in APP/PS1 mice. Analysis of the amount of time spent in the exact target area demonstrated that there was no significant difference between any of the APP/PS1 groups in spatial acuity (Figure 16).

### Example 11

### Chronic effects of treatment on reversal learning and memory

Average daily performance of wild-type mice during the acquisition phase of the reversal water maze task is illustrated in Figure 17. Unlike the Morris water maze, escape latency in the reversal training phase, was significantly affected by drug treatment (p=0.001). Tukey's multiple comparisons test indicated that there was a significant overall difference in escape latency between wild-type saline and wild-type NAcGIP[Lys(37)PAL]-treated mice. Two-way repeated measures ANOVA demonstrated that escape latency (Figure 17A) significantly decreased over time, as expected (p<0.0001). In line with escape latency, path length (Figure 17B) was significantly different overall between wild-type treatment groups, (p=0.0004), and decreased over time, as expected (p<0.0001). Dunnett's multiple comparisons post-hoc test revealed that the path length to the escape platform of wild-type liraglutide (p<0.01), wild-type NAcGIP[Lys(370PAL] (p<0.0001), and wild-type Xenin-25[Lys(13)PAL]-treated mice (p<0.05) was significantly reduced compared with wild-type saline treated mice. Overall, speed was also significantly altered by drug treatment (p=0.0263), but was not affected by training day (p=0.1276). Dunnett's multiple comparisons test revealed a significant difference between swimming speed of wild-type saline and wild-type liraglutide-treated mice (Figure 17C).

Spatial memory was assessed in a reversal probe trial conducted 24 hours after the final training session on day 5 (Figure 18). In wild-type saline-treated mice a one-way ANOVA indicated that there was a significant difference between quadrants (p<0.0037). Dunnett's multiple comparisons post-hoc test demonstrated that saline-treated wild-type mice spent significantly more time in the target quadrant than the CCW (p<0.05), OPP (p<0.01) and CW (p<0.01) quadrants, indicating intact reversal memory. Liraglutide-treated wild-type mice demonstrated a significant difference between quadrants (p=0.0008). Post-hoc analyses demonstrated that liraglutide-treated wild-type mice spent significantly more time in the target quadrant than the CCW (p<0.01), OPP (p<0.001) and CW (p<0.05) quadrants. NAcGIP[Lys(37)PAL]-treated wild-type mice also demonstrated a significant difference between quadrants (p<0.0001). Dunnett's post-hoc test demonstrated that NAcGIP[Lys(37)PAL]-treated wild-type mice spent significantly more time in the target quadrant than the CCW (p<0.001), OPP (p<0.0001) and CW (p<0.01) quadrants. Xenin-25[Lys(13)PAL]-treated wild-type mice also demonstrated a significant difference between quadrants (p=0.0017). Dunnett's multiple comparisons post-hoc test demonstrated that Xenin-25[Lys(13)PAL]-treated wild-type mice spent significantly more time in the target quadrant than the OPP quadrant (p<0.001). Together this demonstrates that spatial memory of wild-type mice is apparently minimally affected by chronic treatment with liraglutide, NAcGIP[Lys(37)PAL] or Xenin-25[Lys(13)PAL].

Spatial acuity was assessed by measuring the amount of time spent swimming at the precise location where the escape platform had been located (Figure 19). There were no significant differences observed between wildtype treatment groups.

Average daily performance of APP/PS1 mice during the acquisition phase of the reversal water maze task is illustrated in Figure 20. An overall two-way repeated measures ANOVA demonstrated that escape latency (Figure 20A) in APP/PS1 mice was significantly impacted by drug treatment (p<0.0001), as well as reducing significantly overtime (p<0.0001). Dunnett's multiple comparisons test highlighted an overall difference in escape latency between the APP/PS1 liraglutide (p<0.0001), APP/PS1 NAcGIP[Lys(37)PAL] (p<0.05) and APP/PS1 Xenin-25[Lys(13)PAL]-treated mice (p<0.001), when compared with APP/PS1 saline-treated controls. An overall repeated measures two-way ANOVA of path length demonstrated that significant differences were associated with drug (p=0.0364) and time (p<0.0001). Tukey's multiple comparisons test demonstrated that path length was not significantly different in APP/PS1 liraglutide, NAcGIP[Lys(37)PAL], or Xenin-25[Lys(13)PAL]-treated mice compared with APP/PS1 saline-treated controls. A significant difference between APP/PS1 liragalutide and NAcGIP[Lys(37)PAL]-treated mice (p<0.05) was identified. Upon analysis of swim speed, an overall repeated measures two-way ANOVA demonstrated that there was a significant impact of drug treatment on swim speed in the reversal water maze training in APP/PS1-treated mice (p=0.0098), and swim speed was significantly different across days (p=0.0497). Tukey's multiple comparisons test demonstrated an overall difference in the swimming speed of APP/PS1 Xenin-25[Lys(13)PAL]-treated mice compared with APP/PS1 saline treated mice (Figure 20C; p<0.05). In the probe trial, analysis of the percentage time spent swimming in each quadrant was used to determine spatial memory. In APP/PS1 saline-treated mice a one-way ANOVA indicated that there was no significant difference between time spent in each of the quadrants (p=0.2292), indicating that spatial memory was impaired. Liraglutide-treated APP/PS1 mice demonstrated a significant difference between quadrants (p<0.0001). Tukey's post-hoc analysis demonstrated that liraglutide-treated mice spent significantly more time in the target quadrant than the CCW (p<0.001), OPP (p<0.0001) and CW (p<0.001) quadrants. One-way ANOVA demonstrated significant variation in time spent in each of the quadrants by NAcGIP[Lys(37)PAL]-treated APP/PS1 mice (p=0.0194). Tukey's multiple comparisons post-hoc test demonstrated that NAcGIP[Lys(37)PAL]-treated APP/PS1 mice spent significantly more time in the target quadrant than the CCW quadrant (p<0.05). Xenin-25[Lys(13)PAL]-treated APP/PS1 mice also demonstrated a significant difference between quadrants (p=0.0073). Dunnett's multiple comparisons post-hoc test demonstrated that Xenin-25[Lys(13)PAL]-treated wild-type mice spent significantly less time in the CCW quadrant than the CW quadrant (p<0.05). Together this demonstrates that liraglutide treatment is associated with improved reversal learning and memory, while NAcGIP[Lys(37)PAL] and Xenin-25[Lys(13)PAL] improve learning but not memory in the reversal water maze task. Analysis of the amount of time spent in the exact target area demonstrated that APP/PS1 liraglutide-treated mice spent significantly more time (p<0.05) in the exact platform location than APP/PS1 saline-treated controls (Figure 21).

### Example 12

### Chronic effects of treatment on locomotor function and anxiety

Path length (Figure 22A) and line crosses (Figure 22B) were, for the most part, similar between treated and untreated groups, with the exception of the APP/PS1 NAcGIP[Lys(37)PAL]-treated mice who displayed significantly increased path length compared with wild-type mice treated with saline (p<0.05), liraglutide (p<0.05), NAcGIP[Lys(37)PAL] (p<0.01) and Xenin-25[Lys(13)PAL] (p<0.001) and APP/PS1 saline-treated mice (p<0.05). Line crosses by APP/PS1 NAcGIP[Lys(37)PAL]-treated mice were significantly increased compared with all groups of wild-type mice (p<0.05-p<0.01). Speed (Figure 22C), rearing behaviour (Figure 22D), number of grooming events (Figure 22E), fecal pellets produced in 5 minutes (Figure 22F) and centre/periphery ratio (Figure 22G) were not significantly different across genotype or treatment groups.

### Example 13

### Chronic effects of treatment on recognition memory

Object recognition memory in response to chronic administration of saline, liraglutide, NAcGIP[Lys(37)PAL] and Xenin-25[Lys(13)PAL] is illustrated in Figure 23. All groups of wild-type mice (A) displayed intact recognition memory and preferentially explored novel object (p<0.05-p<0.001), similar to what was observed at baseline, after acute administration of each of the drugs (Figure 10A). Chronic liraglutide and NAcGIP[Lys(37)PAL]-treatment in APP/PS1 mice, however, restored object recognition memory which was impaired at baseline (Figure 10B). Both liraglutide (p<0.001) and NAcGIP[Lys(37)PAL]-treated mice (p<0.05) spent significantly more time exploring the novel over the familiar object. APP/PS1 saline and Xenin-25[Lys(13)PAL] treated mice did not spend significantly more time exploring the novel object.

### Example 14

### Immunohistochemistry

### Aβ plaque load

As illustrated in Figure 25, a Kruskal-Wallis analysis of Aβ plaque load demonstrated significant differences between groups (p<0.0001). Dunn's multiple comparisons post-hoc test revealed that plaque load was significantly reduced by liraglutide (Figure 25B; 39% reduction), NAcGIP[Lys(37)PAL] (Figure 25C; 27% reduction) and Xenin-25[Lys(13)PAL] treatment (Figure 25D; 29% reduction) when compared with APP/PS1 saline-treated mice (Figure 25A; p<0.0001). APP/PS1 liraglutide-treated mice also had a significantly reduced plaque load compared with APP/PS1 NAcGIP[Lys(37)PAL]-treated mice (p<0.05). Representative images of congo red staining and subsequent quantification are illustrated in Figure 26. In line with the significant reductions in Aβ plaque load, dense core congophilic plaques were also highly significantly reduced by liraglutide (Figure 26B; 43% reduction), NAcGIP[Lys(37)PAL] (Figure 26C; 45% reduction) and Xenin-25[Lys(13)PAL] treatment (Figure 26D; 57% reduction) when compared with APP/PS1 saline-treated mice (Figure 26A; p<0.0001). Xenin-25[Lys(13)PAL] treatment demonstrated significantly more efficacy than liraglutide in reducing dense core congophilic plaque load (p<0.05).

### Microglia

Representative micrographs illustrating Iba1 staining in the cerebral cortex (Figure 27) and dentate gyrus (Figure 28) of treated wild-type and APP/PS1 mice are shown. Quantification (Figure 29) revealed significantly increased Iba1 staining in the cortex of saline-treated APP/PS1 mice, compared with saline-treated wildtypes (Figure 29A; p<0.0001), whereas APP/PS1 mice treated with liraglutide had significantly reduced Iba1 staining in the cortex (Figure 29F; p<0.0001), compared with APP/PS1 mice treated with saline (Figure 29E). In the cortex of APP/PS1 mice that received NAcGIP[Lys(37)PAL], Iba1 staining was significantly increased (Figure 29G; p<0.0001), compared with liraglutide-treated APP/PS1 mice. Iba1 levels were significantly increased in the dentate gyrus of APP/PS1 saline treated mice (Figure 29E; p<0.0001), compared with saline-treated wild-type controls (Figure 29A). APP/PS1 mice treated with liraglutide had significantly lower Iba1 levels in the dentate gyrus (Figure 29F; p<0.01), compared with APP/PS1 saline controls. Iba1 immunopositivity was also significantly increased in the dentate gyrus of NAcGIP[Lys(37)PAL]-treated APP/PS1 mice (Figure 30G; p<0.0001), compared with APP/PS1 mice that were treated with saline. Treatment with Xenin-25[Lys(13)PAL] for 10 weeks had no significant effect on Iba1 levels in either brain region (Figure 29), indicating that Xenin-25[Lys(13)PAL] treatment had no effect on levels of microglia in the cortex (Figure 27H) or dentate gyrus (Figure 28H) of APP/PS1 mice.

### Astrocytes

Representative micrographs illustrating GFAP staining in the cerebral cortex (Figure 30) and dentate gyrus (Figure 31) of treated wild-type and APP/PS1 mice are shown. Quantification (Figure 32) revealed significantly increased GFAP staining in the cortex of saline-treated APP/PS1 mice, compared with saline-treated wildtypes (Figure 32A; p<0.0001), whereas APP/PS1 mice treated with liraglutide had significantly lower GFAP levels in the cortex (Figure 32F; p<0.0001), compared with APP/PS1 mice that received saline (Figure 30E). Quantification of GFAP immunopositivity in the dentate gyrus (Figure 31B) showed that, compared with saline-treated wild-types (Figure 31A), APP/PS1 mice that received saline had significantly increased GFAP staining (Figure 31E; p<0.05). GFAP staining in the dentate gyrus was not significantly affected by any of the treatments in APP/PS1 mice (Figure 32).

### Neurogenesis

There were significantly enhanced levels of neurogenesis in wild-type Xenin-25[Lys(13)PAL]-treated mice (Figure 33D) compared with saline-treated wild-type (Figure 33A; p<0.001) and APP/PS1 mice (Figure 33E; p<0.0001) and APP/PS1 mice treated with liraglutide (Figure 33F; p<0.0001) and NAcGIP[Lys(37)PAL] (Figure 33G; p<0.0001). Wild-type liraglutide-treated mice (Figure 33B) had enhanced neurogenesis compared with APP/PS1 mice treated with saline (p<0.001), liraglutide (p<0.01) and NAcGIP[Lys(37)PAL] (p<0.05). Wild-type NAcGIP[Lys(37)PAL]-treated mice displayed enhanced neurogenesis compared with APP/PS1 saline (p<0.01) and APP/PS1 liraglutide-treated (p<0.05) mice. APP/PS1 Xenin-25[Lys(13)PAL)-treated mice (Figure 33H) displayed enhanced neurogenesis compared with APP/PS1 saline (p<0.01) and APP/PS1 liraglutide treated mice (p<0.05).

### Synapse density

Representative micrographs of synaptophysin staining in wild-type (Figure 35) and APP/PS1 (Figure 36) groups are illustrated. Quantification of levels of synaptophysin (Figure 37) demonstrates that wild-type mice had significantly elevated synaptophysin optical density than APP/PS1 mice in all layers of hippocampus (Figures 37A-F, p<0.05-p<0.0001). Synaptophysin staining in all groups of wild-type mice was significantly greater than APP/PS1 saline-treated mice in the interior (G) and exterior (H) cortex, while levels in liraglutide, NAcGIP[Lys(37)PAL] and Xenin-25[Lys(13)PAL]-treated mice were equivalent to wild-type controls. APP/PS1 liraglutide-treated animals had significantly higher synaptophysin levels than saline-treated APP/PS1 mice in the polymorphic layer (A; p<0.01), molecular layer (C; p<0.05), stratum pyramidale (E; p<0.0001) and the interior (G; p<0.0001) and exterior (H; p<0.0001) cortex. NAcGIP[Lys(37)PAL]-treated APP/PS1 mice had significantly higher expression levels than saline-treated APP/PS1 mice in the polymorphic layer (A; p<0.0001), the stratum pyramidale (E; p<0.0001) and the interior (G; p<0.0001) and exterior (H; p<0.0001) cortex. Xenin-25[Lys(13)PAL]-treated APP/PS1 mice had significantly higher synaptophysin expression levels than saline-treated APP/PS1 mice in the polymorphic layer (A; p<0.05), the stratum pyramidale (E; p<0.01), the interior (G; p<0.0001) and exterior (H; p<0.0001) cortex.

### Soluble Aβ

As illustrated in Figure 38A, total brain APP levels were unchanged by 10 weeks administration of liraglutide, NAcGIP[Lys(37)PAL] and Xenin-25[Lys(13)PAL], analysed by one-way ANOVA (p=0.3981), when compared with APP/PS1 saline treated controls. Conversely, one-way ANOVA uncovered a highly significant difference between treatment groups in levels of soluble Aβ oligomers (Figure 38B; (p=<0.0001). Tukey's multiple comparisons test demonstrated that Aβ oligomer levels were significantly reduced by liraglutide (p<0.05), NAcGIP[Lys(37)PAL] (p<0.0001) and Xenin-25[Lys(13)PAL] (p<0.0001) treatment compared with saline-treated controls. Additionally NAcGIP[Lys(37)PAL] (p<0.05) and Xenin-25[Lys(13)PAL] treatment (p<0.05) were associated with superior reductions in soluble oligomer levels compared with those observed in response to liraglutide treatment. Brain levels of Aβ1-40 (Figure 38C) were also significantly altered in response to treatment (p=0.0012), indicated by Kruskal-Wallis one-way ANOVA. Dunn's multiple comparisons post-hoc test revealed a significant increase in Aβ1-40 levels in liraglutide-treated APP/PS1 mice compared with APP/PS1 mice treated with saline (p<0.01) and Xenin-25[Lys(13)PAL] (p<0.01). An ordinary one-way ANOVA illustrated that there were no significant differences in levels of Aβ1-42 (Figure 38D) in response to treatment (p=0.2419). Amyloid-β (Aβ)42/40 ratio was significantly impacted by treatment of APP/PS1 mice (p=0.0056, Kruskal-Wallis ANOVA). Dunn's multiple comparisons test revealed a significant reduction in APP/PS1 liraglutide-treated mice compared with APP/PS1 saline treated controls (p<0.01).

The present disclosure demonstrates the effects of 8-10 weeks treatment with liraglutide, NAcGIP[Lys(37)PAL] and Xenin-25[Lys(13)PAL], long-lasting analogues of gut hormones glucagonlike peptide-1, glucose-dependent insulinotropic peptide and xenin-25, respectively, in the APP/PS1 mouse model of Alzheimer's disease. Cognitive function was measured using novel object recognition and Morris water maze tasks. Effects of treatment on soluble and insoluble amyloid-β burden, inflammation, synapse density and neurogenesis was assessed in the brains of APP/PS1 and wild-type mice. Liraglutide and NAcGIP[Lys(37)PAL] improved cognitive performance in APP/PS1 mice and, along with Xenin-25[Lys(13)PAL], reduced amyloid-β burden in the brain. Liraglutide ameliorated inflammation in the brain and synapse density was restored in the hippocampus and cortex of APP/PS1 mice following 10 weeks treatment with liraglutide, NAcGIP[Lys(37)PAL] and Xenin-25[Lys(13)PAL]. Additionally, Xenin-25[Lys(13)PAL] treatment increased numbers of doublecortin-positive neurons in the dentate gyrus of wild-type and APP/PS1 mice, suggesting that this peptide augmented neurogenesis. These results underscore the neuroprotective effects of liraglutide and also provide novel evidence that NAcGIP[Lys(37)PAL] and Xenin-25[Lys(13)PAL] possess neuroprotective properties.

Chronic liraglutide treatment in APP/PS1 mice improved spatial learning in the acquisition phase of the Morris water maze and spatial memory was also restored following liraglutide treatment. Chronic treatment with liraglutide, NAcGIP[Lys(37)PAL] and Xenin-25[Lys(13)PAL] also restored reversal learning in APP/PS1 mice, whereas reversal memory recall was improved by liraglutide only. These beneficial effects on cognitive function appear to be related to the positive effects of liraglutide on synaptic plasticity. The finding that liraglutide improves learning and memory is further supported by results depicted here, which demonstrate heightened performance in the reversal water maze task by liraglutide-treated APP/PS1 mice. In addition, time spent in the exact target area in the reversal probe trial, a measure of spatial acuity, was significantly elevated in liraglutide-treated APP/PS1 mice, highlighting the robust effect of liraglutide on cognitive performance. The present disclosure illustrates that reversal learning was also improved in APP/PS1 mice treated with NAcGIP[Lys(37)PAL] and Xenin-25[Lys(13)PAL].

The present disclosure provides further evidence that NAcGIP[Lys(37)PAL] also has a positive effect, albeit subtle, on cognitive function in APP/PS1 mice. Data illustrated here indicate that Xenin-25[Lys(13)PAL] enhanced reversal learning in cognitively impaired APP/PS1 mice. Since Xenin-25[Lys(13)PAL] exerts its effects through NTR1, the improvement in reversal learning depicted here, likely represents activation of central NTR1 by Xenin-25[Lys(13)PAL]. The cognitive flexibility that successful learning of the reversal water maze demands, relies on activity within several brain regions, including the entorhinal cortex, hippocampus and prefrontal cortex, areas in which NTR1 is expressed. Modulation of NTR1 in the hippocampus and cortical areas could explain the enhanced reversal learning illustrated here. Impaired recognition memory was apparent in saline-treated APP/PS1 mice and was restored following chronic treatment with liraglutide. Similarly, NAcGIP[Lys(37)PAL] treatment also restored recognition memory in APP/PS1 mice.

Ten weeks liraglutide treatment reduced Aβ immunopositivity and dense core plaque number in APP/PS1 mice. A significant reduction of Aβ deposition and plaque number was detected in NAcGIP[Lys(37)PAL] and Xenin-25[Lys(13)PAL]-treated APP/PS1 mice. Results depicted in the current disclosure may represent the culmination of a sequence of events initiated by activation of neuronal NTR1 in the brain by Xenin-25[Lys(13)PAL], enhanced intracellular calcium and stimulation of phospholipases C and A2, activation of PKC, enhancement of α-secretase APP cleavage and subsequent reduction of Aβ production and deposition.

The immunohistochemical data described above is supported by the biochemical data, which demonstrate reductions of soluble Aβ oligomers in the brains of APP/PS1 mice treated with liraglutide, NAcGIP[Lys(37)PAL] and Xenin-25[Lys(13)PAL]. Furthermore, treatment with liraglutide reduced the Aβ42/40 ratio. Since Aβ42 levels were no different between treatment groups, the altered Aβ42/40 ratio is most likely due to a relative increase in non-toxic Aβ40 in liraglutide-treated animals. Together, this suggests that liraglutide treatment reduced Aβ oligomers and plaque deposition by modifying APP processing, whereas NAcGIP[Lys(37)PAL] and Xenin-25[Lys(13)PAL] treatment reduced amyloid burden via alternative mechanisms. One possibility is that NAcGIP[Lys(37)PAL] enhanced Aβ clearance and degradation, a proposition that is supported by the finding that levels of microglia were increased in the brains of NAcGIP[Lys(37)PAL]-treated APP/PS1 mice. Given that microglia are known to actively engulf and degrade Aβ, it is not unreasonable to suggest that the reduction of Aβ deposition and soluble oligomer load shown here, is due to increased activation and mobilisation of phagocytic microglia.

Neurogenesis was increased in the dentate gyrus of wild-type and APP/PS1 mice treated with Xenin-25[Lys(13)PAL]. In the present disclosure, liraglutide and NAcGIP[Lys(37)PAL] moderately enhanced the number of doublecortin-positive neurons in the dentate gyrus, however the differences were not significant compared to saline-treated controls. This shows that while liraglutide and NAcGIP[Lys(37)PAL] increased the number of immature neurons in the dentate gyrus, hippocampal neurogenesis was robustly enhanced following Xenin-25[Lys(13)PAL] treatment in wild-type and APP/PS1 mice; a previously unreported finding.

The reduction in hippocampal and cortical synapse density that was apparent in APP/PS1 mice was ameliorated in the polymorphic layer and stratum pyramidale of the hippocampus and in the cerebral cortex of APP/PS1 mice treated with liraglutide, NAcGIP[Lys(37)PAL] and Xenin-25[Lys(13)PAL].

The current disclosure treated 7 month-old APP/PS1 mice for 10 weeks, which increased synaptophysin staining in the polymorphic layer, molecular layer and stratum pyramidale, as well as the cerebral cortex, suggesting that these particular areas of the brain contain synapses that are more readily modified by liraglutide. Since synaptophysin levels remained low in the granule cell layer, stratum radiatum and stratum oriens of treated APP/PS1 mice, these results might also reflect increased vulnerability of synapses in these areas to the synaptotoxic effects associated with Aβ pathology. NAcGIP[Lys(37)PAL] treatment in the present disclosure increased synapse density in the polymorphic layer, stratum pyramidale and the cortex of APP/PS1 mice. The present disclosure also demonstrates, for the first time, that Xenin-25[Lys(13)PAL] treatment in APP/PS1 mice increased synaptophysin staining in the polymorphic layer, stratum pyramidale and cerebral cortex. Results of the present disclosure demonstrate, for the first time, that Xenin-25[Lys(13)PAL] improves cognitive function, enhances neurogenesis and synapse density and ameliorates Aβ pathology in the APP/PS1 mouse model of AD. This disclosure has shown that liraglutide robustly ameliorates cognitive dysfunction and neuropathology in APP/PS1 mice. NAcGIP[Lys(37)PAL] and Xenin-25[Lys(13)PAL] were also shown to mitigate soluble and insoluble amyloid burden and modestly improved cognitive function in APP/PS1 mice.

## Claims

1. A peptide analogue of xenin for use in the treatment of neurological disorders, wherein the peptide analogue comprises xenin and at least one amino acid substitution or modification comprising addition of a fatty acid selected from the group comprising a C-8 octanoyl group, a C-10 decanoyl group, a C-12 lauroyl group, a C-14 myristoyl group, a C-16 palmitoyl group, a C-18 stearoyl group, and a C-20 acyl group.

2. A peptide analogue for use according to Claim 1, wherein the at least one amino acid modification comprises the addition of a C-16 palmitoyl group.

3. A peptide analogue for use according to Claim 1 or 2, wherein the or each fatty acid is attached at a lysine residue.

4. A peptide analogue for use according to any one of Claims 1-3, wherein the peptide analogue comprises xenin and a C-16 palmitoyl group attached at the lysine residue at position 13 of the peptide analogue.

5. A peptide analogue for use according to any preceding claim, wherein the peptide analogue further comprises at least one amino acid modification comprising attachment of a polymer moiety of the general formula HO-(CH₂-O-CH₂)ₙ-H, wherein n is an integer between 1 and 22.

6. A composition for use in the treatment of neurological disorders, wherein the composition comprises a peptide analogue of xenin, wherein the peptide analogue comprises xenin and at least one amino acid substitution or modification comprising addition of a fatty acid selected from the group comprising a C-8 octanoyl group, a C-10 decanoyl group, a C-12 lauroyl group, a C-14 myristoyl group, a C-16 palmitoyl group, a C-18 stearoyl group, and a C-20 acyl group, and a pharmaceutically acceptable carrier.

7. A peptide analogue for use according to any one of Claims 1-5, or a composition for use according to Claim 6, wherein use comprises administration of a pharmaceutically effective amount of the peptide analogue or the composition.

8. A peptide analogue or a composition for use according to Claim 7, wherein use comprises administration of 0.25 - 25.00 nmol/kg body weight of the peptide analogue or an equivalent amount of the composition.

9. A peptide analogue for use according to any one of Claims 1-5, 7, and 8, or a composition for use according to any one of Claims 6-8, wherein the neurological disorder is a disorder selected from the group of disorders affecting cognitive function; and dysfunctional cognitive processes.

10. A peptide analogue for use according to any one of Claims 1-5, 7, and 8, or a composition for use according to any one of Claims 6-8, wherein the neurological disorder is a neurodegenerative disease.

11. A peptide analogue for use according to any one of Claims 1-5, 7, and 8, or a composition for use according to any one of Claims 6-8, wherein the neurological disorder is Alzheimer's disease (AD).

## Patentansprüche

1. Peptidanalogon von Xenin zur Verwendung bei der Behandlung von neurologischen Störungen, wobei das Peptidanalogon Xenin und mindestens eine Aminosäuresubstitution oder -modifikation umfasst, umfassend ein Hinzufügen einer Fettsäure ausgewählt aus der Gruppe umfassend eine C-8-Octanoylgruppe, eine C-10-Decanoylgruppe, eine C-12-Lauroylgruppe, eine C-14-Myristoylgruppe, eine C-16-Palmitoylgruppe, eine C-18-Stearoylgruppe und eine C-20 Acylgruppe.

2. Peptidanalogon zur Verwendung nach Anspruch 1, wobei die mindestens eine Aminosäuremodifikation das Hinzufügen einer C-16-Palmitoylgruppe umfasst.

3. Peptidanalogon zur Verwendung nach Anspruch 1 oder 2, wobei die oder jede Fettsäure an einen Lysinrest gebunden ist.

4. Peptidanalogon zur Verwendung nach einem der Ansprüche 1-3, wobei das Peptidanalogon Xenin und eine am Lysinrest an Position 13 des Peptidanalogons gebundene C-16-Palmitoylgruppe umfasst.

5. Peptidanalogon zur Verwendung nach einem der vorhergehenden Ansprüche, wobei das Peptidanalogon ferner mindestens eine Aminosäuremodifikation umfasst, die eine Bindung einer Polymergruppierung der allgemeinen Formel HO-(CH₂-O-CH₂)ₙ-H umfasst, wobei n eine ganze Zahl zwischen 1 und 22 ist.

6. Zusammensetzung zur Verwendung bei der Behandlung von neurologischen Störungen, wobei die Zusammensetzung ein Peptidanalogon von Xenin, wobei das Peptidanalogon Xenin und mindestens eine Aminosäuresubstitution oder -modifikation umfasst, umfassend ein Hinzufügen einer Fettsäure ausgewählt aus der Gruppe umfassend eine C-8-Octanoylgruppe, eine C-10-Decanoylgruppe, eine C-12-Lauroylgruppe, eine C-14-Myristoylgruppe, eine C-16-Palmitoylgruppe, eine C-18-Stearoylgruppe und eine C-20-Acylgruppe, und einen pharmazeutisch verträglichen Träger umfasst.

7. Peptidanalogon zur Verwendung nach einem der Ansprüche 1-5 oder Zusammensetzung zur Verwendung nach Anspruch 6, wobei die Verwendung eine Verabreichung einer pharmazeutisch wirksamen Menge des Peptidanalogons oder der Zusammensetzung umfasst.

8. Peptidanalogon oder Zusammensetzung zur Verwendung nach Anspruch 7, wobei die Verwendung eine Verabreichung von 0,25 - 25,00 nmol/kg Körpergewicht des Peptidanalogons oder einer äquivalenten Menge der Zusammensetzung umfasst.

9. Peptidanalogon zur Verwendung nach einem der Ansprüche 1-5, 7 und 8 oder Zusammensetzung zur Verwendung nach einem der Ansprüche 6-8, wobei die neurologische Störung eine Störung ausgewählt aus der Gruppe von Störungen, welche die kognitive Funktion beeinträchtigen; und dysfunktionale kognitive Prozesse ist.

10. Peptidanalogon zur Verwendung nach einem der Ansprüche 1-5, 7 und 8 oder Zusammensetzung zur Verwendung nach einem der Ansprüche 6-8, wobei die neurologische Störung eine neurodegenerative Erkrankung ist.

11. Peptidanalogon zur Verwendung nach einem der Ansprüche 1-5, 7 und 8 oder Zusammensetzung zur Verwendung nach einem der Ansprüche 6-8, wobei die neurologische Störung die Alzheimer-Krankheit (Alzheimer's disease - AD) ist.

## Revendications

1. Analogue peptidique de la xénine pour une utilisation dans le traitement de troubles neurologiques, dans lequel l'analogue peptidique comprend de la xénine et au moins une substitution ou une modification d'acide aminé comprenant l'ajout d'un acide gras choisi dans le groupe comprenant un groupe octanoyle en C8, un groupe décanoyle en C10, un groupe lauroyle en C12, un groupe myristoyle en C14, un groupe palmitoyle en C16, un groupe stéaroyle en C18 et un groupe acyle en C20.

2. Analogue peptidique pour une utilisation selon la revendication 1, dans lequel l'au moins une modification d'acide aminé comprend l'ajout d'un groupe palmitoyle en C16.

3. Analogue peptidique pour une utilisation selon la revendication 1 ou 2, dans lequel le ou chaque acide gras est fixé au niveau d'un résidu lysine.

4. Analogue peptidique pour une utilisation selon l'une quelconque des revendications 1 à 3, dans lequel l'analogue peptidique comprend de la xénine et un groupe palmitoyle en C16 fixé au niveau du résidu lysine en position 13 de l'analogue peptidique.

5. Analogue peptidique pour une utilisation selon une quelconque revendication précédente, dans lequel l'analogue peptidique comprend en outre au moins une modification d'acide aminé comprenant la fixation d'un groupement polymère de formule générale HO-(CH₂-O-CH₂)ₙ-H, dans lequel n est un nombre entier entre 1 et 22.

6. Composition pour une utilisation dans le traitement de troubles neurologiques, dans laquelle la composition comprend un analogue peptidique de la xénine, dans laquelle l'analogue peptidique comprend de la xénine et au moins une substitution ou une modification d'acide aminé comprenant l'ajout d'un acide gras choisi dans le groupe comprenant un groupe octanoyle en C8, un groupe décanoyle en C10, un groupe lauroyle en C12, un groupe myristoyle en C14, un groupe palmitoyle en C16, un groupe stéaroyle en C18 et un groupe acyle en C20, et un vecteur pharmaceutiquement acceptable.

7. Analogue peptidique pour une utilisation selon l'une quelconque des revendications 1 à 5, ou une composition pour une utilisation selon la revendication 6, dans lequel (laquelle) l'utilisation comprend l'administration d'une quantité pharmaceutiquement efficace de l'analogue peptidique ou de la composition.

8. Analogue peptidique ou une composition pour une utilisation selon la revendication 7, dans lequel (laquelle) l'utilisation comprend l'administration de 0,25 à 25,00 nmol/kg de poids corporel de l'analogue peptidique ou d'une quantité équivalente de la composition.

9. Analogue peptidique pour une utilisation selon l'une quelconque des revendications 1 à 5, 7 et 8, ou une composition pour une utilisation selon l'une quelconque des revendications 6 à 8, dans lequel (laquelle) le trouble neurologique est un trouble choisi dans le groupe des troubles affectant la fonction cognitive ; et des processus cognitifs dysfonctionnels.

10. Analogue peptidique pour une utilisation selon l'une quelconque des revendications 1 à 5, 7 et 8, ou une composition pour une utilisation selon l'une quelconque des revendications 6 à 8, dans lequel (laquelle) le trouble neurologique est une maladie neurodégénérative.

11. Analogue peptidique pour une utilisation selon l'une quelconque des revendications 1 à 5, 7 et 8, ou une composition pour une utilisation selon l'une quelconque des revendications 6 à 8, dans lequel (laquelle) le trouble neurologique est la maladie d'Alzheimer (MA).


### SEQUENCE LISTING

<110> UNIVERSITY OF ULSTER
<120> Compositions for use in the treatment of neurological disease
<130> P116591PC00
<150> GB 1603510.7
   <151> 2016-02-29
<160> 5
<170> PatentIn version 3.5
<210> 1
   <211> 25
   <212> PRT
   <213> Homo sapiens
<400> 1
   Met Leu Thr Lys Phe Glu Thr Lys Ser Ala Arg Val Lys Gly Leu Ser
   1               5                   10                  15
   Phe His Pro Lys Arg Pro Trp Ile Leu
               20                  25
<210> 2
   <211> 153
   <212> PRT
   <213> Homo sapiens
<400> 2
   Met Val Ala Thr Lys Thr Phe Ala Leu Leu Leu Leu Ser Leu Phe Leu
   1               5                   10                  15
   Ala Val Gly Leu Gly Glu Lys Lys Glu Gly His Phe Ser Ala Leu Pro
               20                  25                  30
   Ser Leu Pro Val Gly Ser His Ala Lys Val Ser Ser Pro Gln Pro Arg
           35                  40                  45
   Gly Pro Arg Tyr Ala Glu Gly Thr Phe Ile Ser Asp Tyr Ser Ile Ala
       50                  55                  60
   Met Asp Lys Ile His Gln Gln Asp Phe Val Asn Trp Leu Leu Ala Gln
   65                  70                  75                  80
   Lys Gly Lys Lys Asn Asp Trp Lys His Asn Ile Thr Gln Arg Glu Ala
                   85                  90                  95
   Arg Ala Leu Glu Leu Ala Ser Gln Ala Asn Arg Lys Glu Glu Glu Ala
               100                 105                 110
   Val Glu Pro Gln Ser Ser Pro Ala Lys Asn Pro Ser Asp Glu Asp Leu
           115                 120                 125
   Leu Arg Asp Leu Leu Ile Gln Glu Leu Leu Ala Cys Leu Leu Asp Gln
       130                 135                 140
   Thr Asn Leu Cys Arg Leu Arg Ser Arg
   145                 150
<210> 3
   <211> 42
   <212> PRT
   <213> Homo sapiens
<400> 3
   Tyr Ala Glu Gly Thr Phe Ile Ser Asp Tyr Ser Ile Ala Met Asp Lys
   1               5                   10                  15
   Ile His Gln Gln Asp Phe Val Asn Trp Leu Leu Ala Gln Lys Gly Lys
               20                  25                  30
   Lys Asn Asp Trp Lys His Asn Ile Thr Gln
           35                  40
<210> 4
   <211> 20
   <212> DNA
   <213> Unknown
<220>
   <223> Artificial Sequence
<400> 4
   gaattccgac atgactcagg 20
<210> 5
   <211> 20
   <212> DNA
   <213> Unknown
<220>
   <223> Artificial Primer
<400> 5
   gttctgctgc atcttggaca 20